# EUROPEAN PATENT APPLICATION

(11) **EP 3 835 328 A1**
(43) Date of publication of application: **16.06.2021**
(21) Application number: 19846718.5
(22) Date of filing: 06.08.2019
(51) Int. Cl.: C08F 2/44, A61K 8/25, A61K 8/29, A61Q 1/12, A61Q 19/00, B01J 13/18, C08F 2/18, C08L 101/00, C09D 7/62, C09D 201/00, C09K 3/00

(54) **ORGANIC INORGANIC COMPOSITE PARTICLE, METHOD FOR PRODUCING SAME, AND APPLICATION THEREOF**

(30) Priority: 09.08.2018 JP 2018150712; 09.08.2018 JP 2018150714; 18.07.2019 JP 2019132979; 19.07.2019 JP 2019133837
(71) Applicant: Sekisui Kasei Co., Ltd., Kita-ku Osaka-shi Osaka 530-8565 (JP)
(72) Inventor: MATSUURA, Haruhiko, Osaka, Osaka 5308565 (JP); MAEYAMA, Yosuke, Osaka, Osaka 530-8565 (JP)
(74) Representative: Müller-Boré & Partner Patentanwälte PartG mbB
(86) International application number: PCT/IB2019/056673
(87) International publication number: WO 2020/031079

(57) **Abstract**

The present invention provides an organic-inorganic composite particle that is excellent in the reflectivity of visible light and near infrared light, and has high light diffusing property and opacifying property, a method for producing the same, and use thereof. More specifically, the present invention relates to an organic-inorganic composite particle comprising an outer shell composed of a crosslinked polymer, and a cavity that is partitioned with the outer shell, wherein the composite particle contains, inside the cavity, a porous structure in which silica particles as a first inorganic particle are interconnected, and a second inorganic particle other than a silica particle, and has a volume average particle diameter of 0.5 to 100 µm, a method for producing the same, and use thereof.

## Description

### TECHNICAL FIELD

The present invention relates to an organic-inorganic composite particle, a method for producing the same, and use thereof. More particularly, the organic-inorganic composite particle of the present invention has a unique shape, and is suitable for uses utilizing its property, such as a cosmetic material, a paint composition, a heat insulating resin composition, a light diffusing resin composition, and a light diffusion film.

The present application claims priorities based on Japanese Patent Application No. 2018-150712 filed on August 9, 2018, Japanese Patent Application No. 2018-150714 filed on August 9, 2018, Japanese Patent Application No. 2019-132979 filed on July 18, 2019, and Japanese Patent Application No. 2019-133837 filed on July 19, 2019, the contents of which are incorporated herein by reference.

### BACKGROUND TECHNOLOGY

Conventionally, in uses such as a cosmetic material, a paint composition, a heat insulating resin composition, a light diffusing resin composition, and a light diffusion film, in order to impart modification of tactile sensation, the soft focus effect, the matting property, and the light diffusing property or the like, an inorganic particle such as a resin particle, a silica particle, a glass particle, titanium oxide, aluminum oxide, and calcium carbonate are used as an additive.

As a specific additive, for example, a hollow resin particle has been proposed (see Patent Documents 1 and 2).

In addition, a method for obtaining a microcapsule particle encapsulating single or plural silica particles, by preparing a microcapsule particle encapsulating a silica precursor by applying a method of synthesizing a hollow particle of the micron size, and thereafter, performing a sol-gel reaction has been proposed (see Non-Patent Document 1).

However, there has been a problem that the hollow resin particle of Patent Documents 1 and 2 and the microcapsule particle encapsulating single or plural silica particles of Non-Patent Document 1, for example, cannot be said to be sufficient in the light diffusing property due to an internal space, and are insufficient for obtaining high light diffusing property and opacifying property, and the excellent reflectivity of visible light and near infrared light when added to a resin composition such as paint.

Then, the present applicant has proposed that an organic-inorganic composite particle, an outer shell of which is composed of a crosslinked polymer, and which contains silica of a porous structure inside a capsule can solve the above-mentioned problem (see Patent Document 3).

### PRIOR ART DOCUMENTS

### Patent Documents

Patent Document 1: Japanese Unexamined Patent Application, First Publication No. 2009-237342
Patent Document 2: International Publication WO 2014/030754
Patent Document 3: International Publication WO 2017/150423

### Non-Patent Documents

Non-Patent Document 1: Polymer Preprints, Japan Vol. 64, No. 2 (2015) 1R11 (Preparation of silica-including microcapsule by sol-gel reaction in polymer capsule, Suzuki et al.)

### SUMMARY OF THE INVENTION

### PROBLEM TO BE SOLVED BY THE INVENTION

However, although the organic-inorganic composite particle of Patent Document 3 has high light diffusing property, it has been desired to provide an organic-inorganic composite particle having higher light diffusing property. In addition, in Patent Document 3, as a pore making agent for forming a porous structure composed of a silica particle inside a capsule, the organic-inorganic composite particle is produced using an organic solvent, but from a view point of influence on a manufacturer at the time of producing, it is desirable that the organic solvent is used as little as possible. For that reason, it has been desired that a method for producing an organic-inorganic composite particle without using an organic solvent is provided.

Then, an object of the present invention is to provide an organic-inorganic composite particle that is excellent in the reflectivity of visible light and near infrared light, and has high light diffusing property and opacifying property, a method for producing the same, and use thereof.

### MEANS FOR SOLVING THE PROBLEM

The present inventors have found that when in an organic-inorganic composite particle, an outer shell of which is composed of a crosslinked polymer, and which contains silica of a porous structure inside a capsule, a particle consisting of an inorganic substance other than silica is further contained inside the capsule, the above-mentioned problem can be solved. In addition, the present inventors have also found that by adding an inorganic thickener to a monomer mixture, an organic-inorganic composite particle can be produced without using an organic solvent, leading to the present invention.

Specifically, according to the present invention, there is provided an organic-inorganic composite particle including an outer shell composed of a crosslinked polymer, and a cavity that is partitioned with the outer shell, wherein the composite particle contains, inside the cavity, a porous structure in which silica particles as a first inorganic particle are interconnected, and a second inorganic particle other than a silica particle, and has a volume average particle diameter of 0.5 to 100 µm.

In addition, according to the present invention, there is provided a method for producing an organic-inorganic composite particle, the method being a method for producing the organic-inorganic composite particle, the method including steps of: forming an outer shell composed of a crosslinked polymer, and a cavity that is partitioned with the outer shell, by suspension polymerizing a mixture comprising 100 parts by weight a radical polymerizable monofunctional monomer, 20 to 150 parts by weight of a crosslinkable monomer, 60 to 400 parts by weight of silicon alkoxide as a silica precursor, and 0.1 to 10 parts by weight of a second inorganic particle, in the presence of a radical polymerization initiator, in an aqueous medium, and forming a porous structure in which silica particles are interconnected inside the cavity, by gelling silicon alkoxide after formation of the outer shell or simultaneously with formation of the outer shell.

Furthermore, according to the present invention, there is provided a method for producing an organic-inorganic composite particle, including steps of: forming an outer shell composed of a crosslinked polymer, by suspension polymerizing a mixture comprising a radical polymerizable monofunctional monomer and crosslinkable monomer, silicon alkoxide as a silica precursor, and an inorganic thickener, in presence of a radical polymerization initiator and in absence of an organic solvent, in an aqueous medium, and forming a porous structure in which silicon particles are interconnected inside the outer shell, from silicon alkoxide, after formation of the outer shell or simultaneously with formation of the outer shell.

In addition, according to the present invention, there is provided a cosmetic material comprising the organic-inorganic composite particle.

Furthermore, according to the present invention, there is provided a paint composition comprising the organic-inorganic composite particle.

In addition, according to the present invention, there is provided a heat insulating resin composition comprising the organic-inorganic composite particle.

Furthermore, according to the present invention, there is provided a light diffusing resin composition comprising the organic-inorganic composite particle.

In addition, according to the present invention, there is provided a light diffusion film comprising the organic-inorganic composite particle.

### EFFECTS OF INVENTION

According to the present invention, there can be provided an organic-inorganic composite particle that is excellent in the reflectivity of visible light and near infrared light, and exerts high light diffusing property and opacifying property, as well as a cosmetic material, a paint composition, a heat insulating resin composition, a light diffusing resin composition, and a light diffusion film, each of which comprises the relevant organic-inorganic composite particle.

In addition, in any of the following cases, there can be provided an organic-inorganic composite particle that exerts the prominent effect of the more excellent light diffusing property and opacifying property, and the reflectivity of near infrared light.
(1) The second inorganic particle has a refractive index of 1.8 or more.
(2) The second inorganic particle has a particle diameter of 0.001 to 3 µm, which is measured by a dynamic light scattering method.
(3) The first inorganic particle and the second inorganic particle have 5 to 50% by weight based on the total weight of an organic-inorganic composite particle, and imparts a hollow structure to a cavity.
(4) The second inorganic particle is a particle selected from titanium oxide, zirconium oxide, cerium oxide, zinc oxide, niobium oxide, and zirconium silicate.

Furthermore, in the following case, an organic-inorganic composite particle that exerts the prominent effect of the more excellent light diffusing property and opacifying property, and the reflectivity of near infrared light can be more conveniently produced.

(1) The gelling is performed using, as a catalyst, an acid or a base in a cavity that is partitioned with the outer shell, the acid or the base is generated by external stimulation of a latent pH adjusting agent by energy radiation or heat, and the latent pH adjusting agent exists in the cavity, by dissolving the latent pH adjusting agent in a mixture at the suspension polymerization.

In addition, in any of the following cases, an organic-inorganic composite particle that is more excellent in the reflectivity of visible light and near infrared light, and has higher light diffusing property can be more conveniently produced, without using an organic solvent.
(1) The mixture has a viscosity of 0.90 mPa ·s or more at 25°C.
(2) The inorganic thickener is silicic anhydride or a clay mineral.
(3) The porous structure in which silica particles are interconnected shows inclusion of a carbon component in EDX measurement.
(4) The inorganic thickener is a hydrophobic silica particle that is silicic anhydride, and the hydrophobic silica particle has a specific surface area by a BET method of 15 to 330 m²/g.
(5) The organic-inorganic composite particle has a volume average particle diameter of 0.5 to 100 µm.
(6) The porous structure has 5 to 50% by weight based on the total weight of an organic-inorganic composite particle.
(7) The hydrophobic silica particle is contained at 0.5 to 100 parts by weight, based on 100 parts by weight of a mixture.
(8) The mixture comprises 100 parts by weight of a monofunctional monomer, 20 to 150 parts by weight of the crosslinkable monomer, and 60 to 400 parts by weight of the silica precursor.

### BRIEF DESCRIPTION OF THE DRAWINGS

Fig. 1 is a surface photograph, a cross-sectional photograph, and a mapping view by SEM-EDS of an organic-inorganic composite particle of Example 1.
Fig. 2 is a surface photograph and a cross-sectional photograph of an organic-inorganic composite particle of Example 2.
Fig. 3 is a surface photograph and a cross-sectional photograph of an organic-inorganic composite particle of Example 3.
Fig. 4 is a surface photograph and a cross-sectional photograph of an organic-inorganic composite particle of Example 4.
Fig. 5 is a surface photograph and a cross-sectional photograph of an organic-inorganic composite particle of Comparative Example 1.
Fig. 6 is a surface photograph and a cross-sectional photograph of an organic-inorganic composite particle of Comparative Example 2.
Fig. 7 is a graph showing light reflectivity for each wavelength of coated films containing various particles in reflection property assessment of ultraviolet, visible, and near infrared lights.
Fig. 8 is a view showing one example of an analysis region set as an interior porous part in a photograph of a particle cross section.
Fig. 9 is a surface photograph and a cross-sectional photograph of an organic-inorganic composite particle of Example 5.
Fig. 10 is a surface photograph and a cross-sectional photograph of an organic-inorganic composite particle of Example 6.
Fig. 11 is a surface photograph and a cross-sectional photograph of an organic-inorganic composite particle of Example 7.
Fig. 12 is a surface photograph and a cross-sectional photograph of an organic-inorganic composite particle of Example 8.
Fig. 13 is a surface photograph and a cross-sectional photograph of an organic-inorganic composite particle of Example 10.
Fig. 14 is a surface photograph and a cross-sectional photograph of an organic-inorganic composite particle of Example 11.
Fig. 15 is a surface photograph and a cross-sectional photograph of an organic-inorganic composite particle of Example 12.
Fig. 16 is a cross-sectional photograph of an organic-inorganic composite particle of Example 14.
Fig. 17 is a cross-sectional photograph of an organic-inorganic composite particle of Example 15.
Fig. 18 is a surface photograph and a cross-sectional photograph of an organic-inorganic composite particle of Comparative Example 3.
Fig. 19 is a graph showing light reflectivity for each wavelength of coated films containing various particles in reflection property assessment of ultraviolet, visible, and near infrared lights.

### BEST MODE FOR CARRYING OUT THE INVENTION

The present invention will be described in detail below. In addition, the present invention is not limited to embodiments described herein, and various modifications can be performed in the scope not departing from the spirit of the present invention.

### (Organic-Inorganic Composite Particle)

The organic-inorganic composite particle of the present invention (hereinafter, also referred to as "composite particle") includes an outer shell composed of a crosslinked polymer. Furthermore, the composite particle may include a cavity that is partitioned with the outer shell. In addition, the composite particle contains a porous structure in which silica particles (hereinafter, also referred to as "first inorganic particle") are interconnected in the outer shell or the interior of the cavity. Furthermore, the composite particle may contain a particle consisting of an inorganic substance other than silica (hereinafter, also referred to as "second inorganic particle"), in the outer shell or the interior of the cavity. In addition, the composite particle has a volume average particle diameter of 0.5 to 100 µm. The composite particle may be referred to as composite fine particle.

### (1) Outer Shell

A kind of a crosslinked polymer is not particularly limited, as long as the polymer can constitute an outer shell. Examples of the crosslinked polymer include polymers derived from a radical polymerizable monomer, and specifically, examples thereof include a copolymer of a monofunctional monomer having one vinyl group and a crosslinkable monomer having two or more vinyl groups.

Examples of the monofunctional monomer having one vinyl group include, for example, alkyl(meth)acrylates having 1 to 16 carbon atoms such as methyl(meth)acrylate, ethyl(meth)acrylate, butyl(meth)acrylate, and cetyl(meth)acrylate; (meth)acrylonitrile, dimethyl maleate, dimethyl fumarate, diethyl fumarate, ethyl fumarate, maleic anhydride, N-vinylcarbazole; styrene-based monomers such as styrene, α-methylstyrene, paramethylstyrene, vinyltoluene, chlorostyrene, ethylstyrene, i-propylstyrene, dimethylstyrene, and bromostyrene, and the like. These monofunctional monomers can be used alone, or by combining a plurality of them.

Examples of the crosslinkable monomer having two or more vinyl groups include, for example, polyfunctional acryl esters such as ethylene glycol di(meth)acrylate, polyethylene glycol di(meth)acrylate, and glycerin tri(meth)acrylate, polyfunctional acrylamide derivatives such as N,N'-methylenebis(meth)acrylamide and N,N'-ethylenebis(meth)acrylamide, polyfunctional allyl derivatives such as diallylamine and tetraallyloxyethane, aromatic divinyl compounds such as divinylbenzene, and the like. These crosslinkable monomers can be used alone, or by combining a plurality of them.

It is preferable that the crosslinkable monomer is contained in the outer shell at a ratio of 20 parts by weight or more, based on 100 parts by weight of the monofunctional monomer. When the content of the crosslinkable monomer is less than 20 parts by weight, the outer shell having the sufficient strength may not be formed. The content is more preferably 20 to 150 parts by weight, further preferably 80 to 120 parts by weight.

### (2) Porous Structure

A porous structure has a configuration that silica particles are interconnected. Herein, the porous structure means a structure that a part of a plurality of silica particles are interconnected, and at unconnected parts, a gap as a macropore is formed between silica particles. It is preferable that the porous structure has a volume in a range of a ratio relative to a total volume of the cavity, described in a column of various physical properties below.

Furthermore, individual silica particles consist mainly of SiO₂. Silica particles can be obtained, for example, by gelling a silica precursor. Examples of the silica precursor include silicon alkoxides having one or more silicon atoms and an alkoxy group (for example, the carbon number 1 to 4) in the same molecule. Specifically, examples thereof include tetraethoxysilane (TEOS), tetramethoxysilane, tetrapropoxysilane, and the like. In addition, examples thereof include oligomers such as a methyl silicate oligomer (manufactured by Mitsubishi Chemical Corporation, product name; MKC Silicate) that is a partially hydrolyzed oligomer of tetramethoxysilane, an ethyl silicate oligomer (manufactured by Tama Chemicals Co., Ltd., product name; Silicate 45 (pentamer), Silicate 48 (decamer)) that is a partially hydrolyzed oligomer of tetraethoxysilane, and a siloxane oligomer. These silica precursors can be used alone, or by combining a plurality of them. Among them, as a monofunctional silica precursor, tetraethoxysilane is preferable, and as a silica precursor that is an oligomer, an ethyl silicate oligomer is preferable.

It is preferable that the porous structure exists in an inner wall of the outer shell, in order to impart the excellent light diffusing property and opacifying property to the composite particle.

It is preferable that the silica precursor is contained in a mixture at a ratio of 60 to 400 parts by weight, based on 100 parts by weight of the monofunctional monomer. When the content of the silica precursor is less than 60 parts by weight, a particle having the sufficient optical performance may not be obtained. When the content is more than 400 parts by weight, since constituents of the outer shell relatively decrease, a particle having the sufficient strength may not be obtained. The content is more preferably 70 to 270 parts by weight, further preferably 80 to 250 parts by weight. In addition, an inclusion ratio of monofunctional monomer-derived components and silica precursor-derived components in the composite particle substantially coincide with the above-mentioned ratio of the monofunctional monomer and the silica precursor.

In addition, the porous structure may contain a crosslinked polymer component, and the crosslinked polymer component may be a crosslinked polymer component that forms the outer shell.

Additionally, the porous structure may contain a carbon component shown in EDX measurement using SEM-EDX.

### (3) Second Inorganic Particle

A second inorganic particle is not particularly limited, as long as it is a particle consisting of an inorganic substance having the composition other than silica. Examples of the second inorganic particle include, for example, particles of titanium oxide, zirconium oxide, cerium oxide, zinc oxide, niobium oxide, zirconium silicate, and the like. Titanium oxide may be surface-treated with alumina, silica or the like. These second inorganic particles can be used alone, or by combining a plurality of them.

It is preferable that the second inorganic particle has a refractive index of 1.8 or more. When a refractive index is less than 1.8, the sufficient light diffusing property improving effect may not be obtained. It is more preferable that a refractive index is 2.0 to 4.0.

Examples of an inorganic particle having a refractive index of 1.8 or more include, for example, particles of titanium oxide, zirconium oxide, cerium oxide, zinc oxide, niobium oxide, zirconium silicate, and the like. The inorganic particle is preferably titanium oxide, zirconium oxide, and cerium oxide.

A method of measuring a refractive index is not particularly limited. For example, examples of the measuring method include the known method (for example, minimum deviation method, critical angle method, V block method or the like) of performing measurement while referring to pages 635 to 640 of "Introduction to Ceramics (Advanced edition), publisher: Uchida Rokakuho Shinsha, publication date: S56 (1981), May 25". A refractive index means a relative refractive index for the air. For measurement, a standard wavelength of 550 nm can be used.

It is preferable that the second inorganic particle has a particle diameter of 0.001 to 3 µm, which is measured by a dynamic light scattering method. When a particle diameter is greater than 3 µm, a particle settles during polymerization, and a desired particle may not be obtained. When a particle diameter is less than 0.001 µm, a particle can be prepared, but the sufficient light reflection performance may not be obtained. It is more preferable that a particle diameter is 0.001 to 1 µm.

The surface of the second inorganic particle may be treated with a surface treating agent, in order to improve the dispersibility in the composite particle. Examples of surface treatment include, for example, water repellency treatment such as silicon treatment, silane coupling treatment, polymer treatment, and the like.

### (4) Weight of First Inorganic Particle and Second Inorganic Particle

It is preferable that a first inorganic particle and a second inorganic particle have 5 to 50% by weight based on the total weight of the composite particle. When the weight of the first inorganic particle and the second inorganic particle is less than 5% by weight, formation of a porous body by silica may be insufficient. When the weight is more than 50% by weight, a ratio of the outer shell relatively reduces, and the sufficient strength may not be possessed. It is more preferable that the weight of these inorganic particles is 10 to 45% by weight.

When the total amount of the first inorganic particle and the second inorganic particle is assumed to be 100 parts by weight, it is preferable that the second inorganic particle is contained in the composite particle in a range of 0.01 to 5 parts by weight. When the content is less than 0.01 part by weight, the sufficient light diffusing property improving effect may not be obtained. When the content is more than 5 parts by weight, a polymerization reaction of a particle may not proceed well. It is more preferable that the content is in a range of 0.05 to 2.5 parts by weight.

The weight of the first inorganic particle and/or the second inorganic particle contained in the composite particle can be measured by fluorescent X-ray measurement.

### (5) Various Physical Properties of Composite Particle

### (a) Volume Average Particle Diameter

It is preferable that the composite particle has a volume average particle diameter of 0.5 to 100 µm. When a volume average particle diameter is less than 0.5 µm, it may be difficult to obtain a fine capsule particle. When a volume average particle diameter is greater than 100 µm, producing may be difficult due to collapse of a capsule particle. Depending on use, a volume average particle diameter is preferably 3 to 80 µm, more preferably 5 to 50 µm.

### (b) Apparent Specific Gravity

When the outer shell is non-porous, it is preferable that the composite particle has the apparent specific gravity of 0.3 to 1.0 g/cm³. When the apparent specific gravity is less than 0.3 g/cm³, a resin layer of the outer shell may be thin, and the strength may reduce. When the apparent specific gravity is greater than 1.0 g/cm³, the effect by a porous structure consisting of silica in the interior may not be sufficiently exerted. It is preferable that the apparent specific gravity is 0.3 to 0.9 g/cm³.

### (c) Outer Shape and the like

An outer shape of the composite particle is not particularly limited, but is preferably near a spherical shape as much as possible.

It is preferable that the thickness of the outer shell is 5 to 40% of a volume average particle diameter. When the thickness of the outer shell is less than 5% of a volume average particle diameter, the outer shell may not have the sufficient strength. When the thickness of the outer shell is greater than 40% of a volume average particle diameter, the effect by a silica structure in the interior may become insufficient. It is more preferable that the thickness of the outer shell is 10 to 30 % of a volume average particle diameter.

The outer shell may be porous. By being porous, as compared with a general silica porous resin particle, improvement in the strength of a particle itself can be expected, and a particle that hardly disintegrates can be provided. In addition, the porosity can also be improved. In addition, a general porous resin particle is made to be porous using a large amount of a pore making agent (solvent), and there is a problem that in order to obtain a fine particle having a great oil absorption amount, it is necessary to use a large amount of a pore making agent, and the productivity remarkably reduces, or the like. In contrast, in the particle of the present invention, the porosity can exceed 90% in a porous structure consisting of silica inside a microcapsule, without using a large amount of a pore making agent. The porous extent can be defined by an oil absorption amount. It is preferable that an oil absorption amount is 150 to 500 ml/100 g. The porous extent can also be defined by another index such as a pore diameter and a pore volume.

It is preferable that the porous structure has 5 to 50% by weight based on the total weight of the composite particle. When the weight of the porous structure is less than 5%, formation of a porous body by silica may become insufficient. When the weight of the porous structure is greater than 50%, a ratio of the outer shell may relatively reduce, and the sufficient strength may not be possessed. It is preferable that the weight of the porous structure is 10 to 45% by weight.

### (Method for Producing Organic-Inorganic Composite Particle)

A method for producing an organic-inorganic composite particle according to a first embodiment of the present invention includes a "polymerization step" of suspension polymerizing a monomer in a mixture comprising a silica precursor, a second inorganic particle, and a radical polymerizable monomer, which are emulsified and dispersed in an aqueous medium, to obtain a microcapsule containing a silica precursor and a second inorganic particle therein, and a "gelling step" of forming a silica particle by gelling the silica precursor in the microcapsule.

### (1) Polymerization Step

In a polymerization step, first, a mixture comprising a silica precursor, a second inorganic particle, and a monomer is dispersed in an aqueous medium by emulsification. In addition, a use amount of the monomer and the content of a monomer-derived component constituting the outer shell are identical substantially.

Regarding the second inorganic particle, a particle itself may be dispersed in an aqueous medium, or a solution in which a particle has been dispersed in a solvent in advance may be dispersed in an aqueous medium. To this solution, a thickener may be added. Examples of the thickener include, for example, organic thickeners such as an acryl-based thickener, a urethane-based thickener, a polyether-based thickener, polyvinyl alcohols, and a cellulose derivative. Examples of an inorganic thickener include a clay mineral. Examples of the clay mineral include smectite clays such as bentonite, montmorillonite, saponite, beidellite, hectorite, stevensite, sauconite, and nontronite, natural clays such as vermiculite, halloysite, swelling mica, zeolite, and attapulgite, or synthetic clays. Only one kind of them may be contained, or two or more kinds may be contained.

Emulsification/dispersion is not particularly limited, and emulsification/dispersion is performed while appropriately adjusting various conditions such as a stirring rate and a stirring time, so that a composite particle of a desired particle diameter is obtained.

It is preferable that polymerization of a monomer is performed in the presence of a radical polymerization initiator. The radical polymerization initiator is not particularly limited, and examples thereof include, for example, persulfates such as ammonium persulfate, potassium persulfate, and sodium persulfate, organic peroxides such as cumene hydroperoxide, di-tert-butyl peroxide, dicumyl peroxide, benzoyl peroxide, lauroyl peroxide, dimethylbis(tert-butylperoxy)hexane, dimethylbis(tert-butylperoxy)hexyne-3, bis(tert-butylperoxyisopropyl)benzene, bis(tert-butylperoxy)trimethylcyclohexane, butyl-bis(tert-butylperoxy)valerate, tert-butyl 2-ethylhexaneperoxyacid, dibenzoyl peroxide, paramenthane hydroperoxide, and tert-butyl peroxybenzoate, and azo compounds such as 2,2'-azobis[2-(2-imidazolin-2-yl)propane] dihydrochloride, 2,2'-azobis[2-(2-imidazolin-2-yl)propane] disulfate dihydrate, 2,2'-azobis(2-amidinopropane) dihydrochloride, 2,2'-azobis[N-(2-carboxyethyl)-2-methylpropionamidine] hydrate, 2,2'-azobis{2-[1-(2-hydroxyethyl)-2-imidazolin-2-yl]propane} dihydrochloride, 2,2'-azobis[2-(2-imidazolin-2-yl)propane], 2,2'-azobis(1-imino-1-pyrrolidino-2-ethylpropane) dihydrochloride,
2,2'-azobis{2-methyl-N-[1,1-bis(hydroxymethyl)-2-hydroxyethyl]propionamide}, 2,2'-azobis[2-methyl-N-(2-hydroxyethyl)propionamide], 4,4'-azobis(4-cyanopentanoic acid), 2,2'-azobisisobutyronitrile, 2,2'-azobis(2-methyl-butyronitrile), 2,2'-azobis(2-isopropylbutyronitrile), 2,2'-azobis(2,3-dimethylbutyronitrile), 2,2'-azobis(2,4-dimethylbutyronitrile), 2,2'-azobis(2-methylcapronitrile), 2,2'-azobis(2,3,3-trimethylbutyronitrile), 2,2'-azobis(2,4,4-trimethylvaleronitrile), 2,2'-azobis(2,4-dimethylvaleronitrile), 2,2'-azobis(2,4-dimethyl-4-ethoxyvaleronitrile), 2,2'-azobis(2,4-dimethyl-4-n-butoxyvaleronitrile), 2,2'-azobis(4-methoxy-2,4-dimethylvaleronitrile), 2,2'-azobis[N-(2-propenyl)-2-methylpropionamide], 2,2'-azobis(N-butyl-2-methylpropionamide), 2,2'-azobis(N-cyclohexyl-2-methylpropionamide), 1,1'-azobis(1-acetoxy-1-phenylethane), 1,1'-azobis(cyclohexane-1-carbonitrile), dimethyl-2,2'-azobis(2-methylpropionate), dimethyl-2,2'-azobisisobutyrate, dimethyl-2,2'-azobis(2-methylpropionate), 2-(carbamoylazo)isobutyronitrile, and 4,4'-azobis(4-cyanovaleric acid). These polymerization initiators can be used alone, or by combining a plurality of them.

It is preferable that the polymerization initiator is contained in the mixture at 0.05 to 5 parts by weight, based on 100 parts by weight of the monomer.

Examples of the aqueous medium include, for example, water, a mixture of water and a water-soluble organic solvent (for example, lower alcohol such as methanol and ethanol), and the like.

Additionally, the polymerization may be performed in the presence of a non-reactive organic solvent. Examples of the non-reactive organic solvent include, for example, pentane, hexane, cyclohexane, heptane, decane, hexadecane, toluene, xylene, ethyl acetate, butyl acetate, methyl ethyl ketone, methyl isobutyl ketone, 1,4-dioxane, methyl chloride, methylene chloride, chloroform, carbon tetrachloride, and the like. These non-reactive organic solvents can be used alone, or by combining a plurality of them.

An addition amount of the non-reactive solvent is not particularly limited, and is 0 to 300 parts by weight, based on 100 parts by weight of the monomer. When the amount exceeds 300 parts by weight, formation of the outer shell may be insufficient.

In the present invention, in order to obtain a composite particle having a non-porous outer shell, the non-reactive organic solvent may be used in a range of 10 to 50 parts by weight, based on 100 parts by weight of the monomer. Depending on a kind of the solvent to be used, when the amount exceeds 50 parts by weight, it becomes easy to obtain a composite particle having a porous outer shell.

Furthermore, a porous structure can be easily formed in a capsule by performing polymerization in the presence of an alkoxide compound of titanium, zirconium or aluminum, which is highly hydrolyzable as compared with silicon alkoxide. When these alkoxide compounds are used, the non-reactive organic solvent may not be used. That is, the present inventors think that since these compounds have higher hydrolyzability than that of the silica precursor such as silicon alkoxide, they have the effect of being gelled in the microcapsule, and inhibiting movement of the silica precursor in the microcapsule, to promote formation of a pore.

Examples of an alkoxide compound of titanium include, for example, isopropyltriisostearoyl titanate, isopropyltristearoyl titanate, isopropyltrioctanoyl titanate, isopropyldimethacrylisostearoyl titanate, isopropyltridodecylbenzenesulfonyl titanate, isopropylisostearoyldiacryl titanate, isopropyl tri(dioctyl phosphate) titanate, isopropyltricumylphenyl titanate, isopropyl tris(dioctyl pyrophosphate) titanate, isopropyl tri(n-aminoethyl-aminoethyl) titanate, tetraisopropyl bis(dioctyl phosphite)titanate, tetraoctyl bis(ditridecylphosphite)titanate, tetra(2,2-diallyloxymethyl-1-butyl)bis(ditridecyl) phosphite titanate, dicumylphenyloxyacetate titanate, bis(dioctylpyrophosphate)oxyacetate titanate, diisostearoylethylene titanate, bis(dioctylpyrophosphate)ethylene titanate, bis(dioctylpyrophosphate)diisopropyl titanate, tetramethyl orthotitanate, tetraethyl orthotitanate, tetrapropyl orthotitanate, tetraisopropyltetraethyl orthotitanate, tetrabutyl orthotitanate, butyl polytitanate, tetraisobutyl orthotitanate, 2-ethylhexyl titanate, stearyl titanate, cresyl titanate monomer, cresyl titanate polymer, diisopropoxy-bis-(2,4-pentadionate)titanium (IV), diisopropyl-bis-triethanolamino titanate, octylene glycol titanate, titanium lactate, acetoacetic ester titanate, diisopropoxybis(acetylacetonato)titanium, di-n-butoxybis(triethanolaluminato)titanium, dihydroxybis(lactato)titanium, titanium-isopropoxy octylene glycolate, tetra-n-butoxytitanium polymer, tri-n-butoxytitanium monostearate polymer, butyl titanate dimer, titanium acetylacetonate, polytitanium acetylacetonate, titanium octylene glycolate, titanium lactate ammonium salt, titanium lactate ethyl ester, titanium triethanol aminate, polyhydroxytitanium stearate, and the like.

Examples of an alkoxide compound of zirconium include zirconium butyrate, zirconium acetylacetonate, acetylacetone zirconium butyrate, zirconium lactate, stearic acid zirconium butyrate, tetra(triethanolamine) zirconate, tetraisopropyl zirconate, and the like.

Examples of an alkoxide compound of aluminum include, for example, acetoalkoxyaluminum diisopropylate, ethyl acetoacetate aluminum diisopropylate, aluminum tris(ethylacetoacetate), alkyl acetoacetate aluminum diisopropylate (the carbon number of alkyl is 1 to 20), aluminum monoacetylacetonate bis(ethylacetoacetate), aluminum tris(acetylacetonate), and the like.

These alkoxide compounds can be used alone, or by combining a plurality of them.

An addition amount of the alkoxide compound is not particularly limited, and is 10 parts by weight or less, based on 100 parts by weight of the monomer. When the addition amounts exceeds 10 parts by weight, since when a monomer mixture is suspended and emulsified in an aqueous medium, the sufficient liquid droplet dispersion safety cannot be retained, a particle may not be obtained.

In addition, herein, when an alkoxide compound of titanium, zirconium or aluminum which is highly hydrolyzable as compared with the non-reactive organic solvent or silicon alkoxide is not added, or when a thickener is not added, single or plural spherical silica particles are generated inside a microcapsule, and a resin particle having a porous structure consisting of silica inside a microcapsule, which is an object of the present invention, cannot be obtained.

Then, an emulsified and dispersed mixture becomes a microcapsule containing a silica precursor in the interior, by subjecting a monomer therein to polymerization. Polymerization is not particularly limited, and it is performed while appropriately adjusting various conditions such as a polymerization temperature and a polymerization time, depending on a kind of a monomer and a polymerization initiator contained in the mixture. For example, a polymerization temperature can be 30 to 80°C, and a polymerization time can be 1 to 20 hours.

### (2) Gelling Step

In a gelling step, by a silica precursor in a microcapsule existing in an emulsified liquid, becoming a silica particle by a gelling reaction, a composite particle is obtained. It is preferable that a gelling reaction is performed while keeping an emulsified liquid alkaline (for example, pH 7 or higher, specifically pH 10 to 14). Keeping of alkalinity can be performed by adding a base such as aqueous ammonia solution, sodium hydroxide, and potassium hydroxide to an emulsified liquid. It is preferable that an addition amount of the base is 1 to 10 equivalents relative to the silica precursor.

A gelling step is not particularly limited, and it can be performed under conditions necessary for the silica precursor to be gelled to become a silica particle (temperature, time, stirring rate, and the like for gelling). For example, a gelling temperature can be 30 to 80°C, and a gelling time can be 1 to 24 hours.

The gelling step may be performed under the coexistence of a latent pH adjusting agent. By the coexistence of the latent pH adjusting agent, it becomes possible to reduce an amount of a base to be added to an emulsified liquid. For example, when ammonia is used as the base, in cases where the latent pH adjusting agent are allowed to coexist, even if an ammonia amount is decreased to 3 equivalents or less (for example, ammonia is not used, 0.01 to 3 equivalents), gelling can be performed effectively. By making it possible to decrease the base, the effect that workability at the time of producing can be improved is exerted. A use amount of the latent pH adjusting agent varies depending on a kind of this agent or producing conditions or the like, and for example, the use amount is preferably 0.01 to 10 parts by weight, based on 100 parts by weight of the silicon precursor. The use amount is more preferably 0.1 to 5 parts by weight.

The latent pH adjusting agent includes a substance that generates an acid or a base by external stimulation such as irradiation of energy radiation and heating. Examples of energy radiation include infrared ray, visible light, and ultraviolet ray, and the like.

Specific examples of the latent pH adjusting agent will be described below.
(i) Examples of the latent pH adjusting agent that generates an acid by heating (thermal acid generator) include, for example, an aryldiazonium salt, a sulfonium salt, an iodonium salt, an ammonium salt, a phosphonium salt, an oxonium salt, an iron-allene complex, an aromatic silanol ammonium complex, diallyliodonium salt-dibenzyloxycopper, an imidazole derivative, a benzylsulfonium salt, a hemiacetal ester, a sulfonic acid ester, and the like.
   Additionally, for example, examples include dicyandiamide, cyclohexyl p-toluenesulfonate, diphenyl(methyl)sulfonium tetrafluoroborate, 4-hydroxyphenylbenzylmethylsulfonium tetrakis(pentafluorophenyl) borate, (4-acetoxyphenyl)benzylmethylsulfonium tetrakis(pentafluorophenyl) borate, 4-hydroxyphenylbenzylmethylsulfonium hexafluoroantimonate, 4-acetoxyphenylbenzylmethylsulfonium hexafluoroantimonate, triphenylsulfonium hexafluoroantimonate, triphenylsulfonium hexafluorophosphonate, di-tert-butylphenyliodonium hexafluorophosphonate, triarylsulfonium hexafluorophosphonate, bis(4-tert-butylphenyl)iodonium hexafluorophosphate, bis(4-fluorophenyl)iodonium trifluoromethanesulfonate, cyclopropyldiphenylsulfonium tetrafluoroborate, diphenyliodonium hexafluoroarsenate, diphenyliodonium trifluoromethanesulfonic acid, 2-(3,4-dimethoxystyryl)-4,6-bis(trichloromethyl)-1,3,5-triazine, 2-[2-(furan-2-yl)vinyl]-4,6-bis(trichloromethyl)-1,3,5-triazine, 4-isopropyl-4'-methyldiphenyliodonium tetrakis(pentafluorophenyl)borate, 2-[2-(5-methylfuran-2-yl)vinyl]-4,6-bis(trichloromethyl)-1,3,5-triazine, 2 -(4-methoxyphenyl)-4,6-bis(trichloromethyl)-1,3,5-triazine, 4-nitrobenzenediazonium tetrafluoroborate, (4-nitrophenyl)(phenyl)iodonium trifluoromethanesulfonate, triphenylsulfonium tetrafluoroborate, triphenylsulfonium bromide, tri-p-tolylsulfonium hexafluorophosphate, tri-p-tolylsulfonium trifluoromethanesulfonate, (1R,2S,5R)-5-methyl-2-(propan-2-yl)cyclohexyl 4-methylbenzenesulfonate, bis[4-n-alkyl(C 10-13)phenyl]iodonium hexafluorophosphate, cyclohexyl 4-methylbenzenesulfonate, and the like.
   In addition, a commercial product may be used. For example, examples include "San-Aid SI-60L, SI-100L, SI-150L" manufactured by Sanshin Chemical Co., Ltd, "TPS", "DBPI" manufactured by Midori Kagaku Co., Ltd., "UVI-6990" manufactured by The Dow Chemical Company, "Irgacure 261" manufactured by Ciba Geigy Corp., and the like.
(ii) Examples of the latent pH adjusting agent that generates a base by heating (thermal base generator) include, for example, 1,2-diisopropyl-3-[bis(dimethylamino)methylene]guadinium 2-(3-benzoylphenyl)propionate, 1,2-dicyclohexyl-4,4,5,5-tetramethylbiguadinium n-butyltriphenylborate, (Z)-{[bis(dimethylamino)methylidene]amino}-N-cyclohexyl(cyclohexylamino)methaneiminiu m tetrakis(3-fluorophenyl)borate, acetophenone O-benzoyloxime, 1,2-bis(4-methoxyphenyl)-2-oxoethyl cyclohexylcarbamate, dimethyl 1,4-dihydro-2,6-dimethyl-4-(2-nitrophenyl)-3,5-pyridinedicarboxylate, 2-nitrobenzyl cyclohexylcarbamate, 1,5,7-triazabicyclo[4.4.0]dec-5-ene 2-(9-oxoxanthen-2-yl)propionate, and the like.
(iii) Examples of the latent pH adjusting agent that generates an acid by irradiation of energy radiation (photoacid generator) include bis(cyclohexylsulfonyl)diazomethane,
   2-methyl-2-[(4-methylphenyl)sulfonyl]-1-[4-(methylthio)phenyl]-1-propanone, bis(tert-butylsulfonyl)diazomethane, bis(4-methylphenylsulfonyl)diazomethane, diphenyl-4-methylphenylsulfonium trifluoromethanesulfonate, diphenyl-2,4,6-trimethylphenylsulfonium p-toluenesulfonate, diphenyl(4-methoxyphenyl)sulfonium trifluoromethanesulfonate, 4-methylphenyldiphenylsulfonium nonafluorobutanesulfonate, tris(4-methylphenyl)sulfonium nonafluorobutanesulfonate, (1R,2S,5R)-5-methyl-2-(propan-2-yl)cyclohexyl 4-methoxybenzenesulfonate, bis(4-tert-butylphenyl)iodonium hexafluorophosphate, bis(4-fluorophenyl)iodonium trifluoromethanesulfonate, cyclopropyldiphenylsulfonium tetrafluoroborate, diphenyliodonium hexafluoroarsenate, diphenyliodonium trifluoromethanesulfonic acid, 2-(3,4-dimethoxystyryl)-4,6-bis(trichloromethyl)-1,3,5-triazine, 2-[2-(furan-2-yl)vinyl]-4,6-bis(trichloromethyl)-1,3, 5-triazine, 4-isopropyl-4'-methyldiphenyliodonium tetrakis(pentafluorophenyl)borate, 2-[2-(5-methylfuran-2-yl)vinyl]-4,6-bis(trichloromethyl)-1,3,5-triazine, 2-(4-methoxyphenyl)-4,6-bis(trichloromethyl)-1,3,5-triazine, 4-nitrobenzenediazonium tetrafluoroborate, (4-nitrophenyl)(phenyl)iodonium trifluoromethanesulfonate, triphenylsulfonium tetrafluoroborate, triphenylsulfonium bromide, tri-p-tolylsulfonium hexafluorophosphate, tri-p-tolylsulfonium trifluoromethanesulfonate, and the like.
   In addition, a commercial product may be used. For example, examples include "San-Aid SI-60L, SI-100L, SI-150L" manufactured by Sanshin Chemical Co., Ltd, "BBI-109", "TPS", "DBPI" manufactured by Midori Kagaku Co., Ltd., "UVI-6990" manufactured by The Dow Chemical Company, "Irgacure 261" manufactured by Ciba Geigy Corp., and the like.
(iv) Examples of the latent pH adjusting agent (photobase generator) that generates a base by irradiation of energy radiation include (Z)-{[bis(dimethylamino)methylidene]amino}-N-cyclohexyl (cyclohexylamino)methaneiminium tetrakis(3-fluorophenyl)borate, 1,2-dicyclohexyl-4,4,5,5-tetramethylbiguadinium n-butyltriphenylborate, 1,2-diisopropyl-3-[bis(dimethylamino)methylene]guadinium 2-(3-benzoylphenyl)propionate, 9-anthrylmethyl N,N-diethylcarbamate, (E)-1-piperidino-3-(2-hydroxyphenyl)-2-propen-1-one, 1-(anthraquinon-2-yl)ethylimidazole carboxylate, 2-nitrophenylmethyl 4-methacryloyloxypiperidine-1-carboxylate, acetophenone O-benzoyloxime, 1,2-bis(4-methoxyphenyl)-2-oxoethyl cyclohexylcarbamate, dimethyl 1,4-dihydro-2,6-dimethyl-4-(2-nitrophenyl)-3,5-pyridinedicarboxylate, 2-nitrobenzyl cyclohexylcarbamate, 1,5,7-triazabicyclo[4.4.0]dec-5-ene 2-(9-oxoxanthen-2-yl)propionate, and the like.

An addition time of the latent pH adjusting agent is not particularly limited, as long as the relevant latent pH adjusting agent exists in the cavity that is partitioned with the outer shell at least at the time of gelling. For example, by dissolving the latent pH adjusting agent in a mixture comprising a silica precursor and a monomer at suspension polymerization, it is allowed to exist in the cavity. When the latent pH adjusting agent is used, a gelling temperature can be 35 to 180°C, and a gelling time can be 0.1 to 48 hours.

### (3) Other Steps

The composite particle after a gelling step can be taken out from an emulsified liquid by passing through centrifugation, water washing, and drying, as necessary.

In addition, a method for producing an organic-inorganic composite particle according to a second embodiment of the present invention includes an "outer shell formation step" of forming an outer shell composed of a crosslinked polymer, by suspension polymerizing a mixture comprising a radical polymerizable monofunctional monomer and crosslinkable monomer, silicon alkoxide as a silica precursor, and an inorganic thickener, in the presence of a radical polymerization initiator and in the absence of an organic solvent, in an aqueous medium, and a "porous structure formation step" of forming a porous structure in which silica particles are interconnected inside the outer shell, from silicon alkoxide, after formation of the outer shell or simultaneously with formation of the outer shell.

### (1) Outer Shell Formation Step

In a mixture, a radical polymerizable monofunctional monomer and crosslinkable monomer, silicon alkoxide as a silica precursor, and an inorganic thickener are contained.

The radical polymerizable monofunctional monomer is, for example, a monomer having one vinyl group, and the radical polymerizable crosslinkable monomer is, for example, a monomer having two or more vinyl groups.

Examples of the radical polymerizable monofunctional monomer include, for example, alkyl (meth)acrylates having 1 to 16 carbon atoms such as methyl (meth)acrylate, ethyl (meth)acrylate, butyl (meth)acrylate, and cetyl (meth)acrylate; (meth)acrylonitrile, dimethyl maleate, dimethyl fumarate, diethyl fumarate, ethyl fumarate, maleic anhydride, N-vinylcarbazole; styrene-based monomers such as styrene, α-methylstyrene, paramethylstyrene, vinyltoluene, chlorostyrene, ethylstyrene, i-propylstyrene, dimethylstyrene, and bromostyrene, and the like. These monofunctional monomers can be used alone, or by combining a plurality of them.

Examples of the radical polymerizable crosslinkable monomer include, for example, polyfunctional acryl esters such as ethylene glycol di(meth)acrylate, polyethylene glycol di(meth)acrylate, and glycerin tri(meth)acrylate, polyfunctional acrylamide derivatives such as N,N'-methylenebis(meth)acrylamide and N,N'-ethylenebis(meth)acrylamide, polyfunctional allyl derivatives such as diallylamine and tetraallyloxyethane, aromatic divinyl compounds such as divinylbenzene, and the like. These linkable monomers can be used alone, or by combining a plurality of them.

It is preferable that the crosslinkable monomer is contained in the mixture at a ratio of 20 to 150 parts by weight, based on 100 parts by weight of the monofunctional monomer. When the content of the crosslinkable monomer is less than 20 parts by weight, the outer shell having the sufficient strength may not be formed. When the content is greater than 150 parts by weight, the outer shell may not be formed. It is more preferable that the content is 80 to 120 parts by weight.

Examples of silicon alkoxide as the silica precursor include silicon alkoxide having one or more silicon atoms and an alkoxy group (for example, the carbon number 1 to 4) in the same molecule. Specifically, examples thereof include tetraethoxysilane (TEOS), tetramethoxysilane, tetrapropoxysilane, and the like. In addition, examples thereof include oligomers such as a methyl silicate oligomer (manufactured by Mitsubishi Chemical Corporation, product name; MKC Silicate) that is a partially hydrolyzed oligomer of tetramethoxysilane, an ethyl silicate oligomer (manufactured by Tama Chemicals Co., Ltd., product name; Silicate 45 (pentamer), Silicate 48 (decamer)) that is a partially hydrolyzed oligomer of tetraethoxysilane, and a siloxane oligomer. These silica precursors can be used alone, or by combining a plurality of them. Among them, as the monofunctional silica precursor, tetraethoxysilane is preferable, and as the silica precursor that is an oligomer, an ethyl silicate oligomer is preferable.

It is preferable that the silica precursor is contained in the mixture at a ratio of 60 to 400 parts by weight, based on 100 parts by weight of the monofunctional monomer. When the content of the silica precursor is less than 60 parts by weight, a particle having the sufficient optical performance may not be obtained. When the content is more than 400 parts by weight, since a constituent of the outer shell relatively reduces, a particle having the sufficient strength may not be obtained. The content is more preferably 70 to 270 parts by weight, further preferably 80 to 250 parts by weight.

The inorganic thickener is not particularly limited, as long as a composite particle can be produced in the absence of an organic solvent. For example, an inorganic thickener that can adjust the viscosity of a mixture at 0.90 mPa ·s or higher at 25°C can be suitably used. By using the mixture in this viscosity range, movement of the silica precursor in a microcapsule that is partitioned with an outer shell can be inhibited, and as a result, the interior of the microcapsule can be promoted to become porous. When the viscosity is less than 0.90 mPa·s, this inhibiting effect becomes insufficient, and a particle in which the interior of the microcapsule was made to be porous may not be obtained. The viscosity is more preferably in a range of 0.9 to 1000 mPa·s.

Examples of the inorganic thickener include silicic anhydride or a clay mineral. Examples of the clay mineral include smectite clays such as bentonite, montmorillonite, saponite, beidellite, hectorite, stevensite, sauconite, and nontronite, natural clays such as vermiculite, halloysite, swelling mica, zeolite, and attapulgite, or synthetic clays. Since these have the effect of enhancing the viscosity of silicon alkoxide in the microcapsule, they can inhibit movement of the silica precursor, and can promote the interior of the microcapsule to become porous. Among them, since dispersion into silicon alkoxide is easy, silicic anhydride is preferable, and a hydrophobic silica particle is more preferable. In addition, hydrophobicity herein means that surface treatment was performed with a hydrophobizing agent such as organic silane or a silicone oil.

Examples of the hydrophobizing agent include, for example, silicone oils such as hexamethyldisilazane, vinyltriethoxysilane, vinyltrimethoxysilane, trimethylsilane, trimethylchlorosilane, trimethylethoxysilane, dimethyldichlorosilane, methyltrichlorosilane, allyldimethylchlorosilane, allylphenyldichlorosilane, benzyldimethylchlorosilane, bromomethyldimethylchlorosilane, α-chloroethyltrichlorosilane, β-chloroethyltrichlorosilane, chloromethyldimethylchlorosilane, triorganosilylmercaptan, trimethylsilylmercaptan, triorganosilyl acrylate, vinylmethylacetoxysilane, dimethylethoxysilane, dimethyldimethoxysilane, diphenyldiethoxysilane, hexamethyldisiloxane, 1,3-divinyltetramethyldisiloxane, 1,3-diphenyltetramethyldisiloxane, dimethylpolysiloxane, methyltrimethoxysilane, phenyltrimethoxysilane, methyltriethoxysilane, phenyltriethoxysilane, n-propyltrimethoxysilane, n-propyltriethoxysilane, hexyltrimethoxysilane, hexyltriethoxysilane, octyltriethoxysilane, decyltrimethoxysilane, hexadecyltriethoxysilane, hexadecyltrimethoxysilane, 3-methacryloxypropylmethyldimethoxysilane, 3-methacryloxypropylmethyldiethoxysilane, 3-methacryloxypropyltrimethoxysilane, 3-methacryloxypropyltriethoxysilane, 1,6-bis(trimethoxysilyl)hexane, trifluoropropyltrimethoxysilane, hydrolyzable group-containing siloxane, an organic silane dimethyl silicone oil such as octamethylcyclotetrasiloxane, an alkyl-modified silicone oil (alkyl has, for example, the carbon number of 1 to 3), a γ-methylstyrene-modified silicone oil, a chlorophenyl silicone oil, a fluorine-modified silicone oil, and a methyl hydrogen silicone oil, and the like.

Surface treatment with the hydrophobizing agent is not particularly limited, and any of the known methods can be used. For example, examples include a method of immersing a silica component into a liquid in which a hydrophobizing agent has been dispersed or dissolved in a medium, and removing the medium, and a method of spraying this liquid to a silica component, and removing the medium.

In addition, it is preferable that the hydrophobic silica particle has a specific surface area by a BET method of 15 m²/g or more. When the specific surface area is less than 15 m²/g, the thickening effect of silicon alkoxide in a microcapsule becomes insufficient, and a silica porous structure may not be formed in a capsule. The specific surface area is preferably 15 to 330 m²/g, further preferably 90 to 290 m²/g. In addition, the specific surface area by a BET method is measured, for example, as per DIN66131.

Examples of the hydrophobic silica particle include hydrophobic fumed silica AEROSIL (product name) series that is sold from EVONIK company (for example, R972, R974, R104, R106, R202, R208, R805, R812, R812S, R816, R7200, R8200, R9200, R711, RY50, NY50, NY50L, RX50, NAX50, RX200, RX300, R504, NX90S, NX90G, RY300, REA90, REA200, RY51, NA50Y, RA200HS, NA50H, NA130K, NA200Y, NX130, RY200, RY200S, RY200L, R709, R976S, and the like), a hydrophobic grade of highly dispersible silica WACKER HDK (product name) that is sold from Asahi Kasei Corporation (for example, H15, H18, H20, H30, and the like), and the like. These hydrophobic silica particles can be used alone, or by combining a plurality of them.

It is preferable that the hydrophobic silica particle is contained in the mixture at a ratio of 0.5 to 100 parts by weight, based on 100 parts by weight of the mixture. When the content of the hydrophobic silica particle is less than 0.5 part by weight, the thickening effect of silicon alkoxide in a microcapsule becomes insufficient, and a silica porous structure may not be formed in a capsule. When the content is more than 100 parts by weight, formation of a microcapsule may become insufficient. The content is more preferably 0.5 to 25 parts by weight, further preferably 2.5 to 15 parts by weight.

In addition, it is presumed that the hydrophobic silica particle is indistinctly mixed with a silica particle derived from silicon alkoxide.

In the outer shell formation step, first, a mixture comprising a silica precursor, a monomer, and an inorganic thickener is dispersed in an aqueous medium by emulsification. In addition, a use amount of the monomer, and the content of a monomer-derived component constituting the outer shell are identical substantially.

Emulsification/dispersion is not particularly limited, and it is performed by appropriately adjusting various conditions such as a stirring rate and a stirring time, so that a composite particle of a desired particle diameter is obtained.

Polymerization of the monomer is performed in the presence of a radical polymerization initiator and in the absence of an organic solvent. The organic solvent herein is, for example, a hydrophobic organic solvent such as pentane, hexane, cyclohexane, pentane, decane, hexadecane, toluene, xylene, ethyl acetate, butyl acetate, methyl ethyl ketone, methyl isobutyl ketone, methyl chloride, methylene chloride, chloroform, and carbon tetrachloride (Hydrophobicity herein means that the solubility in water at 25°C is less than 10 g/100 g (water)). In addition, in this organic solvent, a water-soluble organic solvent of a lower alcohol (for example, methanol, ethanol, and the like) is not included (Water solubility herein means that the solubility in water at 25°C is 10 g/100 g (water) or more).

The above-mentioned radical polymerization initiator and aqueous medium can be used at the same kind and addition amount as those of the above-mentioned producing method of the first embodiment.

Furthermore, a silica porous structure can be easily formed in a capsule, by performing polymerization in the presence of an alkoxide compound of titanium, zirconium or aluminum, which is highly hydrolyzable, as compared with silicon alkoxide. The present inventors think that since these compounds have higher hydrolyzability than that of a silica precursor such as silicon alkoxide, they have the effect of being gelled in the microcapsule, and inhibiting movement of the silica precursor in the microcapsule, to promote formation of a pore.

The alkoxide compound such as an alkoxide compound of titanium, an alkoxide compound of zirconium, and an alkoxide compound of aluminum can be used at the same kind and addition amount as those of the above-mentioned producing method of the first embodiment.

Then, the emulsified and dispersed mixture becomes a microcapsule containing a silica precursor in the interior, by subjecting a monomer therein to polymerization. Polymerization is not particularly limited, and it is performed by appropriately adjusting various conditions such as a polymerization temperature and a polymerization time, depending on a kind of the monomer and the polymerization initiator contained in the mixture. For example, a polymerization temperature can be 30 to 80°C, and a polymerization time can be 1 to 20 hours.

### (2) Porous Structure Formation Step

Since a porous structure formation step is performed according to substantially the same manner as that of "(2) Gelling Step" in the above-mentioned producing method of the first embodiment, explanation is omitted herein.

### (3) Other Steps

A composite particle after the porous structure formation step can be taken out from an emulsified liquid by passing through centrifugation, water washing, and drying, as necessary.

### (Uses)

The composite particle can be used for uses such as a cosmetic material, a paint composition, a heat insulating resin composition, a light diffusing resin composition, and a light diffusion film.

### (1) Cosmetic Material

It is preferable that a cosmetic material contains the composite particle in a range of 1 to 40% by weight.

Examples of the cosmetic material include cleaning cosmetics such as soap, body wash, cleansing cream, and facial scrub, skin lotion, cream, milky lotion, facial masks, facial powders, foundation, lipstick, lip cream, cheek rouge, eye-eyebrow cosmetic, manicure cosmetic, hair washing cosmetic, hair-dyeing material, hair dressing, aromatic cosmetic, tooth paste, bathing agent, antiperspirant, sunscreen product, suntan product, body cosmetic materials such as body powder and baby powder, shaving cream, lotions such as preshave lotion, after shave lotion, and body lotion, and the like.

Additionally, ingredients that are generally used in cosmetic materials can be blended according to the purpose, in such a range that the effect of the present invention is not deteriorated. Examples of such ingredients include, for example, water, a lower alcohol, fat and oil and waxes, a hydrocarbon, a higher fatty acid, a higher alcohol, a sterol, a fatty acid ester, a metal soap, a humectant, a surfactant, a polymer compound, a colorant raw material, a perfume, an antiseptic bactericide, an antioxidant, an ultraviolet absorbing agent, and a special blending ingredient.

Examples of the fat and oil and waxes include an avocado oil, an almond oil, an olive oil, a cacao butter, a beef tallow, a sesame oil, a wheatgerm oil, a safflower oil, a shea butter, a turtle oil, a camellia oil, a persic oil, a castor oil, a grape oil, a macadamia nut oil, a mink oil, an egg yolk oil, Japanese wax, a coconut oil, a rose hip oil, a hardened oil, a silicone oil, an orange raffia oil, carnauba wax, candelilla wax, whale wax, a jojoba oil, montan wax, beeswax, lanolin, and the like.

Examples of the hydrocarbon include liquid paraffin, vaseline, paraffin, ceresin, microcrystalline wax, squalane, and the like. Examples of the higher fatty acid include lauric acid, myristic acid, palmitic acid, stearic acid, oleic acid, behenic acid, undecylenic acid, oxystearic acid, linoleic acid, lanolin fatty acid, and synthetic fatty acid.

Examples of the higher alcohol include lauryl alcohol, cetyl alcohol, cetostearyl alcohol, stearyl alcohol, oleyl alcohol, behenyl alcohol, lanolin alcohol, hydrogenated lanolin alcohol, hexyldecanol, octyldecanol, isostearyl alcohol, jojoba alcohol, decyltetradecanol, and the like.

Examples of the sterol include cholesterol, dihydrocholesterol, phytocholesterol, and the like.

Examples of the fatty acid ester include ethyl linoleate, isopropyl myristate, lanolin fatty acid isopropyl ester, hexyl laurate, myristyl myristate, cetyl myristate, octyldodecyl myristate, decyl oleate, octyldodecyl oleate, hexadecyl dimethyloctanoate, cetyl isooctanoate, decyl palmitate, trimyristic acid glycerin, tri(caprylic / capric acid) glycerin, dioleic acid propylene glycol, triisostearic acid glycerin, triisooctanoic acid glycerin, cetyl lactate, myristyl lactate, diisostearyl malate or cholesteryl isostearate, cyclic alcohol fatty acid esters such as cholesteryl 12-hydroxystearate, and the like.

Examples of the metal soap include zinc laurate, zinc myristate, magnesium myristate, zinc palmitate, zinc stearate, aluminum stearate, calcium stearate, magnesium stearate, zinc undecylenate, and the like.

Examples of the humectant include glycerin, propylene glycol, 1,3-butylene glycol, polyethylene glycol, sodium dl-pyrrolidonecarboxylate, sodium lactate, sorbitol, sodium hyaluronate, polyglycerin, xylit, maltitol, and the like.

Examples of the surfactant include anionic surfactants such as higher fatty acid soap, higher alcohol sulfuric ester, N-acylglutamic acid salt, and phosphoric ester salt, cationic surfactants such as an amine salt and a quaternary ammonium salt, amphoteric surfactants such as betaine type, amino acid type, imidazolin type, and lecithin, and nonionic surfactants such as fatty acid monoglyceride, propylene glycol fatty acid ester, sorbitan fatty acid ester, sugar fatty acid ester, polyglycerin fatty acid ester, and ethylene oxide condensate.

Examples of the polymer compound include natural polymer compounds such as gum arabic, tragacanth gum, guar gum, locust bean gum, karaya gum, irish moss, quince seed, gelatine, shellac, rosin, and cacein, semisynthetic polymer compounds such as carboxymethylcellulose sodium, hydroxyethylcellulose, methylcellulose, ethylcellulose, sodium alginate, ester gum, nitrocellulose, hydroxypropylcellulose, and crystalline cellulose, synthetic polymer compounds such as polyvinyl alcohol, polyvinyl pyrrolidone, sodium polyacrylate, a carboxyvinyl polymer, polyvinyl methyl ether, a polyamide resin, a silicone oil, and resin particles such as a nylon particle, a polymethyl methacrylate particle, a crosslinked polystyrene particle, a silicon particle, a urethane particle, a polyethylene particle, and a silica particle.

Examples of the colorant raw material include inorganic pigments such as iron oxide, ultramarine, Prussian blue, chromium oxide, chromium hydroxide, carbon black, manganese violet, titanium oxide, zinc oxide, talc, kaolin, mica, calcium carbonate, magnesium carbonate, isinglass, aluminum silicate, barium silicate, calcium silicate, magnesium silicate, silica, zeolite, barium sulfate, calcined calcium sulfate (calcined gypsum), calcium phosphate, hydroxyapatite, and ceramic powder, and tar dyes such as azo-based, nitro-based, nitroso-based, xathene-based, quinoline-based, anthraquinoline-based, indigo-based, triphenylmethane-based, phthalocyanine-based, and pyrene-based dyes.

Herein, regarding a powder raw material of the above-mentioned polymer compound or colorant raw material or the like, it may have been surface-treated in advance. As a surface treating method, the previously known surface treatment technology can be utilized. For example, examples include treating methods such as oil solution treatment with a hydrocarbon oil, an ester oil, lanolin or the like, silicone treatment with dimethylpolysiloxane, methyl hydrogen polysiloxane, methylphenylpolysiloxane or the like, fluorine compound treatment with perfluoroalkyl group-containing ester, perfluoroalkylsilane, perfluoropolyether, a polymer having a perfluoroalkyl group or the like, silane coupling agent treatment with 3-methacryloxypropyltrimethoxysilane, 3-glycidoxypropyltrimethoxysilane or the like, titanate coupling agent treatment with isopropyltriisostearoyl titanate, isopropyltris(dioctylpyrophosphate) titanate or the like, metal soap treatment, amino acid treatment with acylglutamic acid or the like, lecitin treatment with hydrogenated egg yolk lecitin or the like, collagen treatment, polyethylene treatment, moisture retaining treatment, inorganic compound treatment, and mechanochemical treatment.

Examples of the perfume include natural perfumes such as a lavender oil, a peppermint oil, and a lime oil, and synthetic perfumes such as ethylphenyl acetate, geraniol, and p-tert-butylcyclohexyl acetate. Examples of the antiseptic bactericide include methylparaben, ethylparaben, propylparaben, benzalkonium, benzethonium, and the like.

Examples of the antioxidant include dibutylhydroxytoluene, butylhydroxyanisole, propyl gallate, tocopherol, and the like. Examples of the ultraviolet absorbing agent include inorganic absorbing agents such as titanium oxide, zinc oxide, cerium oxide, iron oxide, and zirconium oxide, and organic absorbing agents such as benzoic acid-based, paraaminobenzoic acid-based, anthranilic acid-based, salicylic acid-based, cinnamic acid-based, benzophenone-based, and dibenzoylmethane-based absorbing agents.

Examples of the special blending ingredient include hormones such as estradiol, estrone, ethynylestradiol, cortisone, hydrocortisone, and prednisone, vitamins such as vitamin A, vitamin B, vitamin C, and vitamin E, skin astringents such as citric acid, tartaric acid, lactic acid, aluminum chloride, aluminum potassium sulfate, allantoinchlorohydroxyaluminum, zinc paraphenolsulfonate, and zinc sulfate, hair growth promoting agents such as cantharis tincture, capsicum tincture, ginger tincture, swertia herb extract, garlic extract, hinokitiol, carpronium chloride, pentadecanoic acid glyceride, vitamin E, estrogen, and photosensitizer, whitening agents such as magnesium phosphate-L-ascorbate and kojic acid, and the like.

### (2) Paint, Heat insulating, and Light Diffusing Compositions

These compositions contain a binder resin, a UV curable resin, a solvent, and the like, as necessary. As the binder resin, a resin that is soluble in an organic solvent or water, or an emulsion-type aqueous resin that can be dispersed in water can be used.

An addition amount of the binder resin or the UV curable resin and that of the composite particle are different depending on the film thickness of a formed coated film, an average particle diameter of the composite particle, and a coating method. An addition amount of the composite particle is preferably 5 to 50% by weight based on the total of the binder resin (solid content when the emulsion-type aqueous resin is used) and the composite particle. The more preferable content is 10 to 50% by weight, and the further preferable content is 20 to 40% by weight.

Examples of the binder resin include an acryl resin, an alkyd resin, a polyester resin, a polyurethane resin, a chlorinated polyolefin resin, an amorphous polyolefin resin, an acrylic silicone resin, an acrylic urethane resin, a fluorine-based resin, and the like, and examples of the UV curable resin include polyfunctional (meth)acrylate resins such as polyhydric alcohol polyfunctional (meth)acrylate; polyfunctional urethane acrylate resins such as those synthesized from diisocyanate, polyhydric alcohol, and (meth)acrylic acid ester having a hydroxy group, and the like.

As the UV curable resin, a polyfunctional (meth)acrylate resin is preferable, and a polyhydric alcohol polyfunctional (meth)acrylate resin having 3 or more (meth)acryloyl groups in one molecule is more preferable. Examples of the polyhydric alcohol polyfunctional (meth)acrylate resin having 3 or more (meth)acryloyl groups in one molecule specifically include trimethylolpropane tri(meth)acrylate, trimethylolethane tri(meth)acrylate, 1,2,4-cyclohexane tetra(meth)acrylate, pentaglycerol triacrylate, pentaerythritol tetra(meth)acrylate, pentaerythritol tri(meth)acrylate, dipentaerythritol triacrylate, dipentaerythritol pentaacrylate, dipentaerythritol tetra(meth)acrylate, dipentaerythritol hexa(meth)acrylate, tripentaerythritol triacrylate, tripentaerythritol hexaacrylate, and the like, and these may be used alone, or two or more kinds may be used together.

When the UV curable resin is used, usually, a photopolymerization initiator is used together. The photopolymerization initiator is not particularly limited.

Examples of the photopolymerization initiator include, for example, acetophenones, benzoins, benzophenones, phosphine oxides, ketals, α-hydroxyalkylphenones, α-aminoalkylphenones, anthraquinones, thioxanthones, azo compounds, peroxides (see Japanese Unexamined Patent Application, First Publication No. 2001-139663, etc.), 2,3-dialkyldione compounds, disulfide compounds, fluoroamine compounds, aromatic sulfoniums, onium salts, borates, active halogen compound, α-acyloxime ester, and the like.

These binder resins or UV curable resins can be appropriately selected depending on the adherability of the paint to a base material to be painted or environments used or the like.

The solvent is not particularly limited, and it is preferable to use a solvent in which the binder resin or the UV curable resin can be dissolved or dispersed. For example, in the case of an oily paint, examples of the solvent include hydrocarbon-based solvents such as toluene and xylene; ketone-based solvents such as methyl ethyl ketone and methyl isobutyl ketone; ester-based solvents such as ethyl acetate and butyl acetate; ether-based solvents such as dioxane, ethylene glycol diethyl ether, and ethylene glycol monobutyl ether, and the like. In the case of an aqueous paint, water, alcohols, and the like can be used. These solvents may be used alone, or may be used by mixing two or more kinds. The content of the solvent in a coating material is usually around 20 to 60% by weight, based on the composition total amount.

In the composition, the known painted surface adjusting agent, flowability adjusting agent, ultraviolet absorbing agent, light stabilizer, curing catalyst, extender pigment, coloring pigment, metal pigment, mica powder pigment, dye or the like may be contained, as necessary.

A method of forming a coated film using the composition is not particularly limited, and any of the known methods can be used. For example, examples include methods such as a spray coating method, a roll coating method, and a brush coating method, and in order to perform coating on a base material such as a film as a thin layer, examples include a coating reverse roll coating method, a gravure coating method, a die coating method, a comma coating method, and a spray coating method. The composition may be diluted in order to adjust the viscosity, as necessary. Examples of the diluent include hydrocarbon-based solvents such as toluene and xylene; ketone-based solvents such as methyl ethyl ketone and methyl isobutyl ketone; ester-based solvents such as ethyl acetate and butyl acetate; ether-based solvents such as dioxane and ethylene glycol diethyl ether; water; alcohol-based solvents, and the like. These diluents may be used alone, or may be used by mixing two or more kinds.

By performing coating on an arbitrary coating surface such as a base material to prepare a coating film, drying this coating film and, thereafter, curing the coating film as necessary, a coated film can be formed. In addition, the coated film using the paint composition is used by coating it on various base materials, and the base material is not particularly limited to metal, timber, glass, plastic or the like. Alternatively, the coated film can also be used by coating it on a transparent base material such as polyethylene terephthalate (PET), polycarbonate (PC), and acryl.

### (3) Light Diffusion Film

A light diffusion film is a film in which a light diffusion layer derived from the above-mentioned light diffusing composition is formed on the surface of a base material such as glass, and a plastic sheet, a plastic film, a plastic lens, and a plastic panel of polycarbonate (PC), acrylic resin, polyethylene terephthalate (PET), triacetylcellulose (TAC) or the like, or a base material such as a cathode ray tube, a fluorescent display tube, and a liquid crystal display plate. Being different depending on uses, the coated film is formed alone, or formed on a base material in combination with a protective film, a hard coat film, a flattening film, a high refractive index film, an insulating film, an electrically conductive resin film, an electrically conductive metal particle film, an electrically conductive metal oxide particle film, or in other cases, a primer film or the like which is used as necessary. In addition, when used in combination, it is not required that the light diffusion layer is necessarily formed on an outermost surface.

### EXAMPLES

The present invention will be more specifically described below by way of Examples, but the present invention is not limited thereto. First, measuring methods in Examples will be described.

### (Measurement of volume average particle diameter)

A volume average particle diameter of the composite particle was measured with Coulter Multisizer™ 3 (measuring apparatus manufactured by Beckman Coulter Inc.). Measurement was carried out using an aperture that was calibrated according to Multisizer™ 3 user's manual published by Beckman Coulter Inc.

In addition, selection of an aperture used in measurement was appropriately performed such as that when a supposed volume average particle diameter of a particle to be measured is 1 µm or more and 10 µm or less, an aperture having the size of 50 µm is selected, when a supposed volume average particle diameter of a particle to be measured is greater than 10 µm and 30 µm or less, an aperture having the size of 100 µm is selected, when a supposed volume average particle diameter of a particle is greater than 30 µm and 90 µm or less, an aperture having the size of 280 µm is selected, when a supposed volume average particle diameter of a particle is greater than 90 µm and 150 µm or less, an aperture having the size of 400 µm is selected, or the like. When a volume average particle diameter after measurement was different from a supposed volume average particle diameter, an aperture was changed to an aperture having the appropriate size, and measurement was performed again.

In addition, when an aperture having the size of 50 µm was selected, Current (aperture current) was set at -800, and Gain was set at 4, when an aperture having the size of 100 µm was selected, Current (aperture current) was set at -1600, and Gain was set at 2, and when apertures having the size of 280 µm and 400 µm were selected, Current (aperture current) was set at -3200, and Gain was set at 1.

As a sample for measurement, a sample obtained by dispersing 0.1 g of a polymer particle in 10 ml of a 0.1 wt% aqueous nonionic surfactant solution using a touch mixer (manufactured by Yamato Scientific Co., Ltd., "TOUCHMIXER MT-31") and an ultrasound washer (manufactured by VELVO CLEAR Co., Ltd., "ULTRASONICCLEANER VS-150") to obtain dispersion was used. During measurement, the interior of a beaker was mildly stirred to an extent that air bubbles do not enter, and at the time point that about 100,000 particles were measured, measurement was terminated. As a volume average particle diameter of the particle, an arithmetic average in a particle size distribution based on the volume of 100,000 particles was adopted.

### (Measurement of Amount of Inorganic Component in Composite Particle)

After 1.0 g of a composite particle that is a measuring object was measured, the measured composite particle was burned in an electric furnace at 750°C for 30 minutes, and the weight (g) of the remaining residue was measured. Then, the weight (g) of the measured residue was divided by the weight (1.0 g) of a composite particle before measurement, and converted into a percentage to obtain the ignition residue (% by weight) of the composite particle.

### (Weight of Porous Structure in Composite Particle)

After 1.0 g of a composite particle that is a measuring object was measured, the measured composite particle was burned in an electric furnace at 750°C for 30 minutes, and the weight (g) of the remaining residue was measured. Then, the weight (g) of the measured residue was divided by the weight (1.0 g) of a composite particle before measurement, and converted into a percentage to obtain the ignition residue (% by weight) of the composited particle. The resulting ignition residue (% by weight) represented the content of a porous structure in the composite particle.

### (Measurement of Amount of Carbon Atom in Internal Porous Structure)

A composite particle was mixed with a photocurable resin D-800 (manufactured by JEOL Ltd.), and the mixture was irradiated with light to thereby obtain a cured product. Thereafter, the cured product was immersed in liquid nitrogen for 5 minutes, cut, and stuck on a sample stage with a carbon tape with a cross section upward. Platinum deposition treatment (15 mV, 240 seconds, 6.0 Pa, distance between target and sample surface 30 mm) was performed using an "Ion Sputter E-1045 Type" sputtering apparatus manufactured by Hitachi High-Tech Corporation. Then, using a secondary electron detector of "Regulus8230" scanning electron microscope manufactured by Hitachi High-Tech Corporation, a cross section of a particle in a sample was photographed (magnification of particle cross section photograph 5000 times). In addition, an acceleration voltage at observation was 10 kV.

In particle cross section observation, using "X-MaxN150" manufactured by Oxford Instruments KK installed on "Regulus8230" manufactured by Hitachi High-Tech Corporation, elementary analysis was performed. An internal porous part in a particle cross section was arbitrarily set as an analysis area, and the atomic number concentration (%) of a carbon atom, and the atomic number concentration (%) of a silicon atom in the analysis area were measured. In addition, as the atomic number concentration (%) of a carbon atom and the atomic number concentration (%) of a silicon atom, numerical values obtained by removing atoms other than a carbon atom and a silicon atom from observed atoms, and performing re-analysis using software attached to "X-MaxN150" manufactured by Oxford Instruments KK so that the total of the atomic number concentration (%) of a carbon atom and the atomic number concentration (%) of a silicon atom became 100% were used. In addition, an acceleration voltage at analysis was 10 kV. Additionally, resolution in setting of image scan was 1024, a dwell time was 5 µs, and an input signal was SE. Additionally, an energy range, the channel number and collection mode in EDS collection spectrum setting were set at automatic setting, and a process time was set at 6.

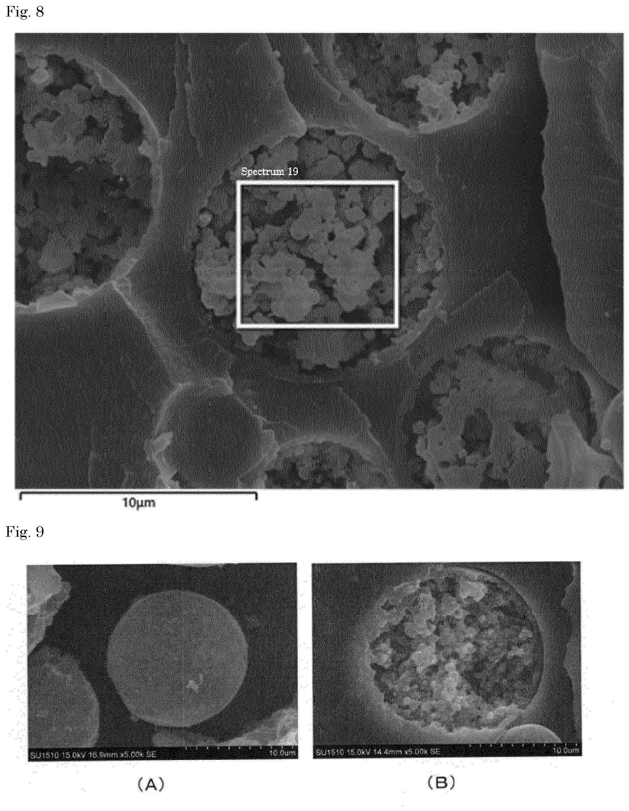

One example of an analysis area that was set as an internal porous part, in a photograph of a particle cross section, is shown in Fig. 8. The interior of a white frame described as spectrum 19 in Fig. 8 was defined as an analysis area. The atomic number concentration (%) of a carbon atom and the atomic number concentration (%) of a silicon atom in a white frame were defined as the atomic number concentration (%) of a carbon atom and the atomic number concentration (%) of a silicon atom in the internal porous structure.

### (Density of Mixed Liquid)

A mixed liquid, a temperature of which had been adjusted with a 25°C constant temperature bath, was added to a 25 mL pycnometer. By dividing the weight (g) of the added mixed liquid by the volume (mL) of the pycnometer, the density of the mixed liquid was obtained.

### (Viscosity of Mixed Liquid)

The viscosity of a mixed liquid, a temperature of which had been adjusted with a 25°C constant temperature bath, was measured using a tuning fork-type vibration type viscometer SV-10 (manufactured by A&D Company, Limited). By dividing a viscosity display value (unit: mPa ·s × g/cm³) on a device by the above-mentioned calculated density of the mixed liquid, the viscosity (unit: mPa ·s) of the mixed liquid was obtained.

### (Particle Surface Observation)

A sample was excised out, an electrically conductive tape was stuck on a sample stage, and the sample was loaded thereon. Using "Osmium Coater Neoc-Pro" coating device manufactured by Meiwafosis Co., Ltd., surface treatment (10 Pa, 5 mA, 10 seconds) of a particle was performed. Then, particle surface appearance of a sample was photographed, using a secondary electron detector of "SU1510" scanning electron microscope manufactured by Hitachi High-Tech Corporation.

### (Particle Cross Section Observation)

After a washing step, a particle that had been dried at 100°C was mixed with a photocurable resin D-800 (manufactured by JEOL Ltd.), and irradiated with ultraviolet light to thereby obtain a cured product. Thereafter, the cured product was cut with a nipper, a cross section part was smoothed using a cutter, and surface treatment (10 Pa, 5 mA, 10 seconds) was performed using an osmium coating device ("Osmium Coater Neoc-Pro" manufactured by Meiwafosis Co., Ltd.). Then, a particle cross section of the sample was photographed using a secondary electron detector of "SU1510" scanning electron microscope manufactured by Hitachi High-Tech Corporation.

Herein, element mapping of a cross section part of the sample was performed with SEM-EDS (Scanning Electron Microscope Energy Dispersive X-ray Spectrometry), and whether silica and titanium oxide exist inside a cavity or not was determined. Specifically, using SEM ("S3400 N" manufactured by Hitachi High-Tech Corporation) and EDS ("EMAXEvolution X-Max" manufactured by HORIBA, Ltd.), uptake at an acceleration voltage 15 KV and 10 frame (800 seconds) was performed, and an element map was obtained by Kα-ray of silicon and Kα-ray of titanium.

### (Particle Diameter of Second Inorganic Particle)

A particle diameter of a second inorganic particle was defined as an average particle diameter measured by utilizing a method called a dynamic light scattering method or a photon correlation method. That is, at 25°C, a dispersed liquid of an inorganic particle in an organic solvent, which had been prepared to 0.1% by volume, was irradiated with laser light, and the intensity of scattered light scattered from the inorganic particle was measured at time change of a microsecond unit. The detected scattering intensity distribution originated from the inorganic particle was fitted to a normal distribution, and a Z average particle diameter was calculated by a cumulant analysis method. This average particle diameter can be simply measured with a measuring device loaded with a commercially available data analysis software, and could be automatically analyzed. In the present Example, the diameter was measured using a particle diameter measuring device "Zetasizer Nano ZS" manufactured by Malvern Instruments, Ltd. (Spectris Co., Ltd.).

### (Surface Treatment Method of Titanium Oxide)

31 g of toluene and 5.0 g of a phosphate-based dispersant as an organic dispersant were weighed, and placed into a mixing tank made of stainless steel, and the mixture was stirred with a mixing machine for 5 minutes. Subsequently, 60 g of titanium oxide as an inorganic particle was additionally placed, and further, the materials were stirred with a mixing machine for 30 minutes. This was named as solution (A). The above-mentioned solution (A) was transferred into a bead mill container filled with zirconia beads having a particle diameter of 1.5 mm, and the interior of the mill was stirred for 60 minutes to disperse the inorganic particle. Then, in order to adjust the viscosity of the dispersion, 3.0 g of bentonite was placed, stirring and dispersion were performed, and a toluene solution of titanium oxide (X) (content of titanium oxide 60.6%, solid content 68.7%) was obtained. A Z average particle diameter of titanium oxide was 780 nm.

### (Example 1)

100 g of methyl methacrylate (MMA) as a monofunctional monomer, 100 g of ethylene glycol dimethacrylate (EGDMA) as a crosslinkable monomer, 160 g of tetraethoxysillane (TEOS) as a silica precursor, 1 g of (1R,2S,5R)-5-methyl-2-(propan-2-yl)cyclohexyl 4-methylbenzenesulfonate (manufactured by Wako Pure Chemical Industries, Ltd.: product name WPAG-699) as a thermal acid generator, 39.75 g of toluene as a non-reactive organic solvent, 2.0 g of 2,2'-azobis(2,4-dimethylvaleronitrile) (manufactured by Wako Pure Chemical Industries, Ltd.; product name V-65) as a polymerization initiator, and 0.77 g of a toluene solution (X) of a titanium oxide particle as a second inorganic particle were mixed and dissolved to prepare a mixture. The resulting mixture was mixed into 1200 g of an aqueous polyvinyl alcohol (PVA) (manufactured by The Nippon Synthetic Chemical Industry Co., Ltd.; product name GOHSENOL GL-5) solution which had been prepared to the concentration of 1.7% by weight.

The resulting mixed liquid was placed into a 2L beaker, and emulsification/dispersion treatment was performed at a rotation number of 5000 rpm for 6 minutes, using a homogenizer (manufactured by CENTRAL SCIENTIFIC COMMERCE, INC.; product name Polytron Homogenizer PT10-35GT). The resulting emulsified liquid was placed into a 2L autoclave made of stainless steel, polymerization for 4 hours was performed at a temperature of 50°C while stirring at 250 rpm with a turbine-like stirring blade having a diameter of 8 cm, and a microcapsule containing TEOS as a silica precursor and titanium oxide in the interior was obtained. Thereafter, by stirring for 2 hours under the conditions of 105°C, progression of a gelling reaction of TEOS in the microcapsule afforded a composite particle.

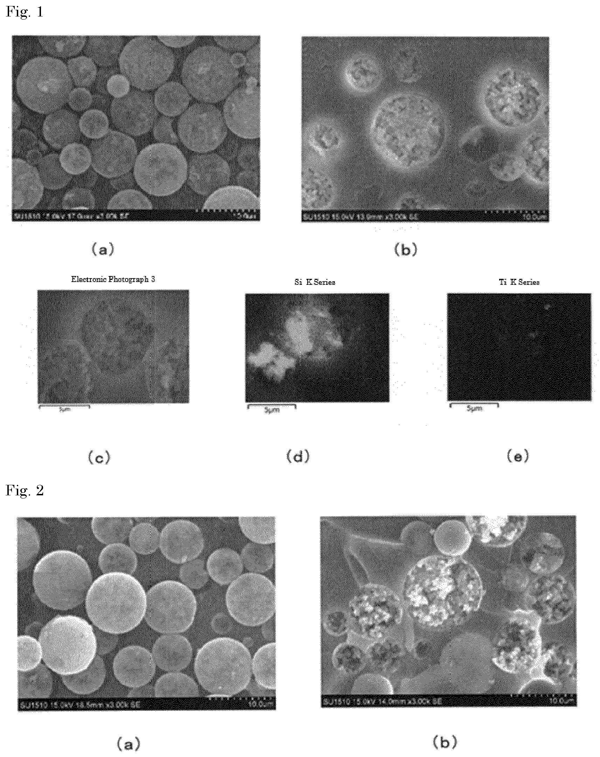

Water washing was repeated, purification was performed, and thereafter, the composite particle was dried overnight with a vacuum oven at 100°C. A surface photograph of the resulting composite particle is shown in Fig. 1 (a), and a cross section photograph is shown in Fig. 1 (b). It could be confirmed that an outer shell composed of a monofunctional monomer and a crosslinkable monomer is formed, and in the interior thereof, a porous structure in which silica particles are interconnected is formed. In addition, a volume average particle diameter was 10.0 µm, and an amount of an inorganic component in the composite particle was 17.9% by weight.

Metal element mapping views by SEM-EDS are shown in Fig. 1 (c) to (e). Fig. 1 (c) is a cross section photograph, Fig. 1 (d) is a view showing existence of silicon, and Fig. 1 (e) is a view showing existence of titanium. From these photographs, existence of silicon and titanium in the composite particle could be confirmed.

### (Example 2)

In the same manner as that of Example 1 except that the toluene solution (X) of a titanium oxide particle as a second inorganic particle was 3.85 g, and toluene of a non-reactive organic solvent was 38.76 g, a composite particle was obtained. A surface photograph of the resulting composite particle is shown in Fig. 2 (a), and a cross section photograph is shown in Fig. 2 (b). It could be confirmed that an outer shell composed of a monofunctional monomer and a crosslinkable monomer is formed, and in the interior thereof, a porous structure in which silica particle are interconnected is formed. In addition, a volume average particle diameter was 10.4 µm, and an amount of an inorganic component in the composite particle was 7.5% by weight.

### (Example 3)

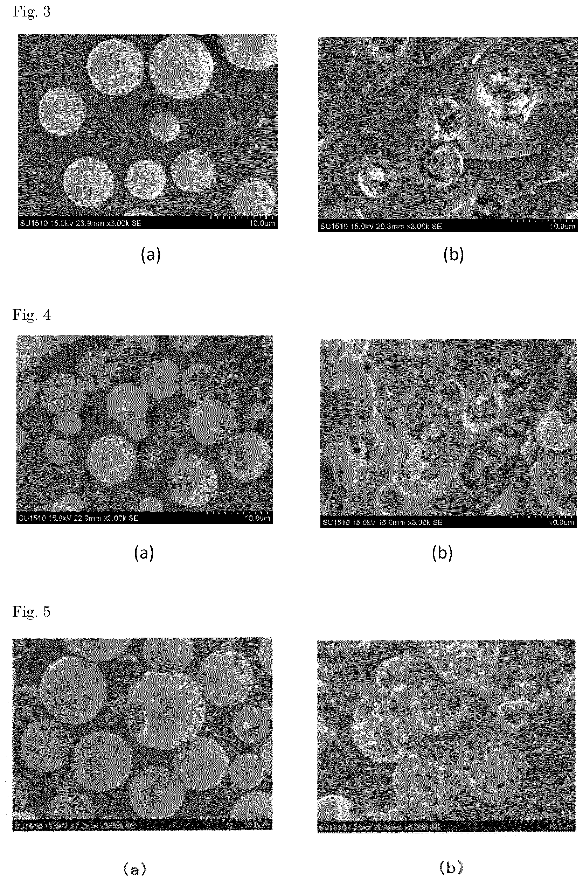

In the same manner as that of Example 1 except that the methanol dispersion of a zirconium oxide particle as a second inorganic component particle was 1.54 g (manufactured by Sakai Chemical Industry Co., Ltd.; product name SZR-M, zirconia content 30% by weight, particle diameter: 3 nm), and toluene as a non-reactive organic solvent was 38.9 g, a composite particle was obtained. A surface photograph of the resulting composite particle is shown in Fig. 3 (a), and a cross section photograph is shown in Fig. 3 (b). It could be confirmed that an outer shell composed of a monofunctional monomer and a crosslinkable monomer is formed, and in the interior thereof, a porous structure in which silica particles are interconnected is formed. In addition, a volume average particle diameter was 11.4 µm, and an amount of an inorganic component in the composite particle was 16.8% by weight.

### (Example 4)

In the same manner as that of Example 1 except that cerium oxide as a second inorganic component particle was 0.46 g (manufactured by Aitec Co., Ltd.; product name CeriaNao particle powder, particle diameter: 10 nm), acetoalkoxyaluminum diisopropionate (manufactured by Ajinomoto Fine-Techno Co., Inc., product name Plenact ALM) as a surface treating agent was 0.046 g, and toluene as a non-reactive organic solvent was 40 g, a composite particle was obtained. A surface photograph of the resulting composite particle is shown in Fig. 4 (a), and a cross section photograph is shown in Fig. 4 (b). It could be confirmed that an outer shell composed of a monofunctional monomer and a crosslinkable monomer is formed, and in the interior thereof, a porous structure in which silica particles are interconnected is formed. In addition, a volume average particle diameter was 11.7 µm, and an amount of an inorganic component in the composite particle was 8.9% by weight.

### (Comparative Example 1)

In the same manner as that of Example 1 except that toluene as a non-reactive organic solvent was 40 g, and a titanium oxide particle was not added, a composite particle was obtained. A surface photograph of the resulting composite particle is shown in Fig. 5 (a), and a cross section photograph is shown in Fig. 5 (b). It could be confirmed that an outer shell composed of a monofunctional monomer and a crosslinkable monomer is formed. In addition, in the interior thereof, a porous structure in which silica particles are interconnected could be confirmed. In addition, a volume average particle diameter was 11.9 µm, and an amount of an inorganic component in the composite particle was 16.0% by weight.

### (Comparative Example 2)

With reference to Non-Patent Document 1, a silica-including particle (composite particle) was prepared. Specifically, 100 g of methyl methacrylate (MMA) as a monofunctional monomer, 100 g of ethylene glycol dimethacrylate (EGDMA) as a crosslinkable monomer, 200 g of tetraethoxysilane (TEOS) as a silica precursor, and 2 g of 2,2'-azobis(4-methoxy-2,4-dimethylvaleronitrile) (manufactured by Wako Pure Chemical Industries, Ltd.; product name V-70) as a polymerization initiator were mixed to prepare a polymerizable composition.

Separately, to 1180 g of ion-exchanged water as an aqueous phase, 20 g of polyvinyl alcohol (PVA) (manufactured by The Nippon Synthetic Chemical Industry Co., Ltd.; GOHSENOL GL-05) was added. The polymerizable composition was placed into this aqueous phase, and emulsification/dispersion treatment was performed at a rotation number of 5000 rpm for 10 minutes, using a homogenizer (manufactured by CENTRAL SCIENTIFIC COMMERCE, INC.; product name Polytron Homogenizer PT10-35GT). The resulting emulsified liquid was placed into a 2L pressure container with a stirring blade, and heating for 24 hours was performed at 30°C while stirring the stirring blade at 350 rpm, to thereby form an outer shell composed of a monofunctional monomer and a crosslinkable monomer. While a reaction temperature and stirring were maintained, aqueous ammonia that is a 10-fold equivalent of TEOS was placed, the mixture was heated for 24 hours, and thereby, a sol-gel reaction of TEOS was allowed to proceed, to thereby obtain an emulsified liquid containing a silica-including particle.

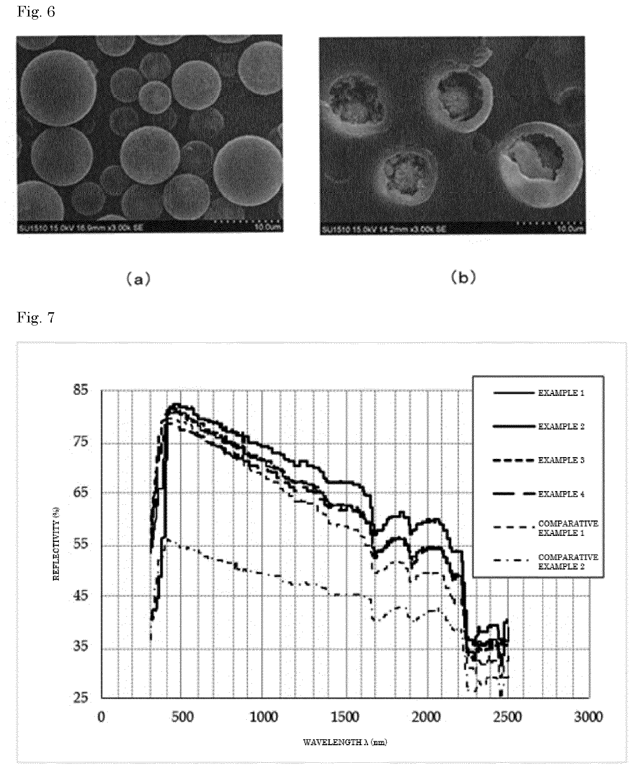

By performing suction filtration on the resulting emulsified liquid, the silica-including particle was taken out from the emulsified liquid. Water washing was repeated, purification was performed, and thereafter, drying was performed in a vacuum oven at 60°C, to thereby obtain a silica-including particle. A surface photograph of the resulting silica-including particle is shown in Fig. 6 (a), and a cross section photograph is shown in Fig. 6 (b). It could be confirmed that an outer shell composed of a monofunctional monomer and a crosslinkable monomer is formed, and in the interior thereof, single or plural silica particles are encapsulated. In addition, a volume average particle diameter was 10.4 µm, and an amount of an inorganic component in the silica-including particle was 22.0% by weight.

Raw materials for producing of silica-including particles of the above-mentioned Examples 1 to 4 and Comparative Examples 1 to 2 and use amounts thereof (parts by weight) are described together in Table 1.

**[Table 1]**

| | | | Example | | | | Comparative Example | |
|---|---|---|---|---|---|---|---|---|
| | | | 1 | 2 | 3 | 4 | 1 | 2 |
| Polymerizable composition | Monofunctional monomer | MMA | 100 | 100 | 100 | 100 | 100 | 80 |
| | Crosslinkable monomer | EGDMA | 100 | 100 | 100 | 100 | 100 | 80 |
| | Polymerization initiator | V-65 | 2 | 2 | 2 | 2 | 2 | - |
| | | V-70 | - | - | - | - | - | 1.6 |
| | Silica precursor | TEOS | 160 | 160 | 160 | 160 | 160 | 160 |
| | Titanium oxide dispersion | | 0.77 | 3.85 | | | - | - |
| | Zirconium oxide dispersion | | | | 1.54 | | | |
| | Cerium oxide | | | | | 0.46 | | |
| | Thermal acid generator | WPAG-699 | 1 | 1 | 1 | 1 | 1 | - |
| | Non-reactive organic solvent | Toluene | 39.75 | 38.76 | 38.90 | 40 | 40 | - |
| | Surface treating agent | Plenact ALM | | | | 0.046 | | |
| Aqueous phase | PVA(GL-05) | | 20 | 20 | 20 | 20 | 20 | 20 |
| | Ion-exchanged water | | 1,180 | 1,180 | 1,180 | 1,180 | 1,180 | 1,180 |
| Amount of second inorganic particle based on 100 parts by weight of monofunctional monomer (parts by weight) | | | 0.46 | 2.33 | 0.46 | 0.46 | - | - |

### (Assessment of Reflection Property of Ultraviolet, Visible, and Near Infrared Lights)

To 10 g of a commercially available aqueous paint (manufactured by ASAHIPEN CORPORATION, product name; Water-based multi use color, Clear), each 2.5 g of composite particles obtained from Examples 1 to 4 and Comparative Examples 1 to 2 was added, and the mixture was defoamed and stirred using a planetary stirring defoaming machine (manufactured by Kurabo Industries Ltd., MAZERUSTAR KK-250), to prepare paint for assessment.

The paint for assessment was coated on a black side of an opacifying ratio test paper with an applicator set at the wet thickness of 250 µm, and thereafter, this was sufficiently dried under room temperature to obtain a sample plate for light reflectivity assessment. Reflectivities for ultraviolet light, visible light, and near infrared light of the sample plate were assessed by the following procedure.

Using an ultraviolet, visible, and near infrared spectrophotometer (Solid Spec3700) manufactured by Shimadzu Corporation as an apparatus for measuring the reflectivity, the reflection property of from ultraviolet light to near infrared light (wavelength 300 to 2500 nm) of a coated surface in the sample plate was measured as reflectivity (%). In addition, measurement was performed using a 60 mm φ integrating sphere, and employing Spectralon as a standard white plate.

In addition, the above-mentioned measurement was performed regarding composite particles of Examples 1 to 4 and Comparative Examples 1 to 2. The obtained results are shown in Fig. 7.

From Fig. 7, it is seen that composite particles of Examples 1 to 4 have higher reflectivity than that of composite particles of Comparative Examples 1 to 2, in almost all wavelengths of from ultraviolet light to near infrared light. Particularly, it is seen that the composite particles have high reflectivity, in a wavelength area of 500 to 2500.

### (Example 5)

A mixture consisting of 80 g of methyl methacrylate (MMA) as a monofunctional monomer, 80 g of ethylene glycol dimethacrylate (EGDMA) as a crosslinkable monomer, 160 g of tetraethoxysilane (TEOS) as a silica precursor, 16 g of hydrophobic fumed silica R972 (EVONIK company, specific surface area by BET method 110±20 m²/g) as a hydrophobic silica particle, 1.6 g of 2,2'-azobis(2,4-dimethylvaleronitrile) (manufactured by Wako Pure Chemical Industries, Ltd.; product name V-65) as a polymerization initiator, and 0.8 g of (1R,2S,5R)-5-methyl-2-propan-2-yl)cyclohexyl 4-methylbenzenesulfonate (manufactured by Wako Pure Chemical Industries, Ltd.; product name WPAG-699) as a thermal acid generator was prepared. The viscosity of the mixture was 3.58 mPa·s.

Separately, to 1280 g of ion-exchanged water, 26 g of magnesium pyrophosphate as a suspension stabilizer, and 0.128 g of lauryl phosphoric acid were added to prepare an aqueous phase. The above-mentioned mixture was placed into this aqueous phase, and emulsification/dispersion treatment for 10 minutes was performed at a rotation number of 7000 rpm, using a homogenizer (manufactured by CENTRAL SCIENTIFIC COMMERCE, INC.; product name Polytron Homogenizer PT10-35). The resulting emulsified liquid was placed into a 2L pressure container with a stirring blade, and heating for 4 hours was performed at 50°C while stirring the stirring blade at 350 rpm, to thereby form an outer shell composed of a monofunctional monomer and a crosslinkable monomer. While stirring was maintained, a reaction temperature was raised to 105°C, heating was performed for 2 hours, and thereby, a gelling reaction of TEOS was made to proceed. Thereafter, by cooling a reaction system to room temperature, an emulsified liquid containing a composite particle was obtained. To the resulting emulsified liquid, hydrochloric acid was added, magnesium pyrophosphate was dissolved, thereafter, suction filtration was performed, and thereby, a composite particle was taken out from the emulsified liquid. Water washing was repeated, purification was performed, and thereafter, drying was performed in a vacuum oven at 60°C, to thereby obtain a composite particle.

A surface photograph of the resulting composite particle is shown in Fig. 9 (A), and a cross section photograph is shown in Fig. 9 (B). It could be confirmed that an outer shell derived from a monofunctional monomer and a crosslinkable monomer is formed, and in the interior thereof, a porous structure in which silica particles are interconnected is formed. In addition, a volume average particle diameter was 20.7 µm, and the weight of a porous structure in the composite particle was 27.0% by weight.

### (Example 6)

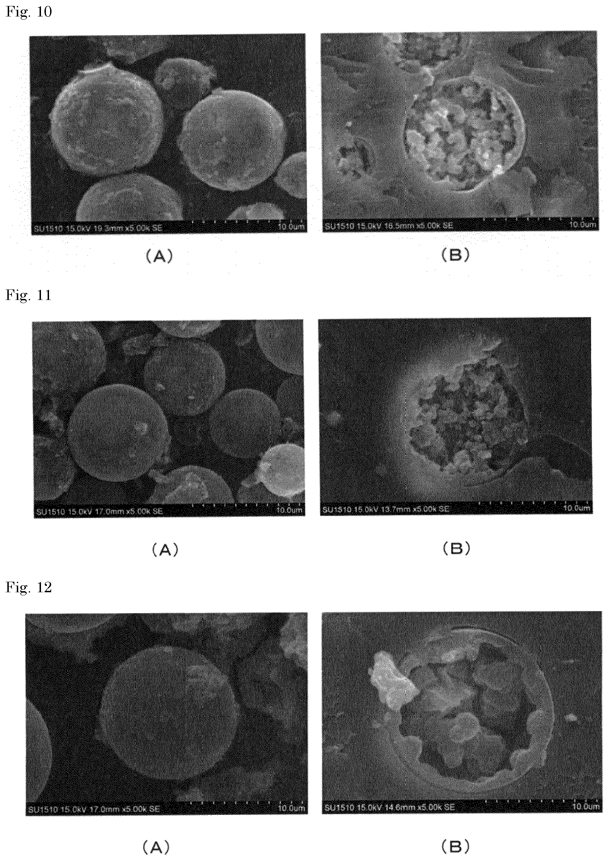

In the same manner as that of Example 5 except that 12 g of a hydrophobic silica particle was used, a composite particle was obtained. In addition, the viscosity of a mixture was 2.39 mPa·s. A surface photograph of the resulting composite particle is shown in Fig. 10 (A), and a cross section photograph is shown in Fig. 10 (B). It could be confirmed that an outer shell composed of a monofunctional monomer and a crosslinkable monomer is formed, and in the interior thereof, a porous structure in which silica particles are interconnected is formed. In addition, a volume average particle diameter was 9.5 µm, and the weight of a porous structure in the composite particle was 24.2% by weight. The weight of silica in the composite particle was 25.1%.

Additionally, when the atomic number concentration (%) of a carbon atom in an internal porous structure was measured, it was 90.4%, and it could be confirmed that a crosslinked polymer component forming an outer shell is contained.

### (Example 7)

In the same manner as that of Example 5 except that 8 g of a hydrophobic silica particle was used, a composite particle was obtained. In addition, the viscosity of a mixture was 1.61 mPa·s. A surface photograph of the resulting composite particle is shown in Fig. 11 (A), and a cross section photograph is shown in Fig. 11 (B). It could be confirmed that an outer shell composed of a monofunctional monomer and a crosslinkable monomer is formed, and in the interior thereof, a porous structure in which silica particles are interconnected is formed. In addition, a volume average particle diameter was 11.4 µm, and the weight of a porous structure in the composite particle was 23.3% by weight.

### (Example 8)

In the same manner as that of Example 5 except that 1.6 g of the hydrophobic silica particle was used, a composite particle was obtained. In addition, the viscosity of a mixture was 0.93 mPa·s. A surface photograph of the resulting composite particle is shown in Fig. 12 (A), and a cross section photograph is shown in Fig. 12 (B). It could be confirmed that an outer shell composed of a monofunctional monomer and a crosslinkable monomer is formed, and in the interior thereof, a porous structure in which silica particles are interconnected is formed. In addition, a volume average particle diameter was 16.1 µm, and the weight of a porous structure in the composite particle was 21.6% by weight.

### (Example 9)

In the same manner as that of Example 5 except that 24 g of the hydrophobic silica particle was used, a composite particle was obtained. In addition, the viscosity of a mixture was 8.07 mPa·s. It could be confirmed that an outer shell composed of a monofunctional monomer and a crosslinkable monomer is formed, and in the interior thereof, a porous structure in which silica particles are interconnected is formed. In addition, a volume average particle diameter was 20.3 µm, and the weight of a porous structure in the composite particle was 29.1% by weight.

### (Example 10)

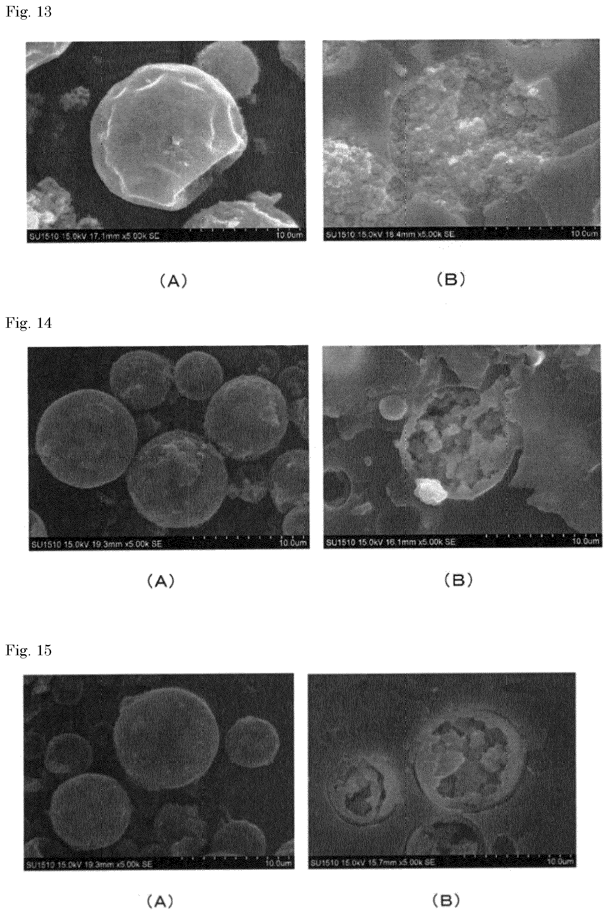

In the same manner as that of Example 5 except that 36 g of the hydrophobic silica particle was used, a composite particle was obtained. In addition, the viscosity of a mixture was 46.3 mPa·s. A surface photograph of the resulting composite particle is shown in Fig. 13 (A), and a cross section photograph is shown in Fig. 13 (B). It could be confirmed that an outer shell composed of a monofunctional monomer and a crosslinkable monomer is formed, and in the interior thereof, a porous structure in which silica particles are interconnected is formed. In addition, a volume average particle diameter was 21.6 µm, and the weight of a porous structure in the composite particle was 31.8% by weight.

### (Example 11)

In the same manner as that of Example 5 except that, as the hydrophobic silica particle, 8 g of hydrophobic fumed silica R974 (EVONIK company, specific surface area by BET method 170±20 m²/g) was used, a composite particle was obtained. In addition, the viscosity of a mixture was 1.76 mPa·s. A surface photograph of the resulting composite particle is shown in Fig. 14 (A), and a cross section photograph is shown in Fig. 14 (B). It could be confirmed that an outer shell composed of a monofunctional monomer and a crosslinkable monomer is formed, and in the interior thereof, a porous structure in which silica particles are interconnected is formed. In addition, a volume average particle diameter was 11.0 µm, and the weight of a porous structure in the composite particle was 23.9% by weight.

### (Example 12)

In the same manner as that of Example 5 except that, as the hydrophobic silica particle, 8 g of hydrophobic fumed silica R976S (EVONIK company, specific surface area by BET method 240±25 m²/g) was used, a composite particle was obtained. In addition, the viscosity of a mixture was 1.54 mPa·s. A surface photograph of the resulting composite particle is shown in Fig. 15 (A), and a cross section photograph is shown in Fig. 15 (B). It could be confirmed that an outer shell composed of a monofunctional monomer and a crosslinkable monomer is formed, and in the interior thereof, a porous structure in which silica particles are interconnected is formed. In addition, a volume average particle diameter was 10.8 µm, and the weight of a porous structure in the composite particle was 24.0% by weight.

### (Example 13)

In the same manner as that of Example 5 except that, as the hydrophobic silica particle, 8 g of hydrophobic fumed silica R812 (EVONIK company, specific surface area by BET method 260±30 m²/g) was used, a composite particle was obtained. In addition, the viscosity of a mixture was 1.27 mPa·s. It could be confirmed that an outer shell composed of a monofunctional monomer and a crosslinkable monomer is formed, and in the interior thereof, a porous structure in which silica particles are interconnected is formed. In addition, a volume average particle diameter was 11.5 µm, and the weight of a porous structure in the composite particle was 24.1% by weight.

### (Example 14)

A mixture consisting of 100 g of methyl methacrylate (MMA) as a monofunctional monomer, 100 g of ethylene glycol dimethacrylate (EGDMA) as a crosslinkable monomer, 200 g of tetraethoxysilane (TEOS) as a silica precursor, 4 g of Sumecton-STN (Kunimine Industries Co., Ltd. organic smectite) as an inorganic thickener, and 2 g of 2,2'-azobis(2,4-dimethylvaleronitrile) (manufactured by Wako Pure Chemical Industries, Ltd.; product name V-65) as a polymerization initiator was prepared. The viscosity of the mixture was 0.91 mPa·s.

Separately, 60 g of polyvinyl alcohol (manufactured by The Nippon Synthetic Chemical Industry Co., Ltd.; GL-05) as a suspension stabilizer was dissolved in 1140 g of ion-exchanged water to prepare an aqueous phase. Into this aqueous phase, the above-mentioned mixture was placed, and emulsification/dispersion treatment for 10 minutes was performed at a rotation number of 5000 rpm, using a homomixer (manufactured by CENTRAL SCIENTIFIC COMMERCE, INC.; product name Polytron Homogenizer PT10-35). The resulting emulsified liquid was placed into a 2L pressure container with a stirring blade, and heating for 4 hours was performed at 50°C while stirring the stirring blade at 350 rpm, to thereby form an outer shell composed of a monofunctional monomer and a crosslinkable monomer. While maintaining stirring, an internal temperature was lowered to 30°C, 65 g of 25% aqueous ammonia (Wako Pure Chemical Industries, Ltd.) was added, this was stirred for 16 hours, thereby, a gelling reaction of TEOS was allowed to proceed, and thereby, an emulsified liquid containing a composite particle was obtained. By performing suction filtration on the resulting emulsified liquid, the composite particle was taken out from the emulsified liquid. Water washing was repeated, purification was performed, and thereafter, drying was performed in a vacuum oven at 60°C, to thereby obtain a composite particle.

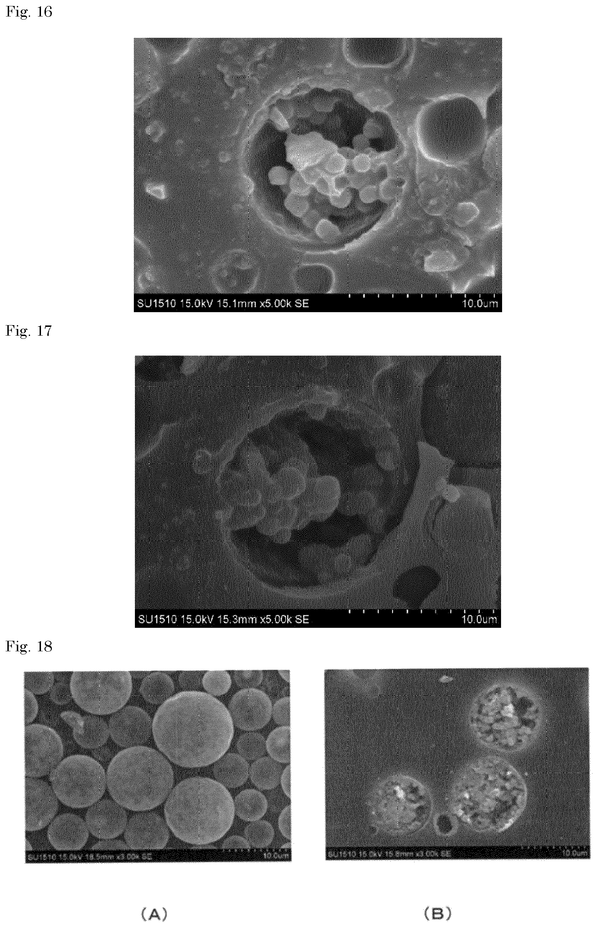

A cross section photograph of the resulting composite particle is shown in Fig. 16. It could be confirmed that an outer shell derived from a monofunctional monomer and a crosslinkable monomer is formed, and in the interior thereof, a porous structure in which silica particles are interconnected is formed. In addition, a volume average particle diameter was 11.5 µm, and the weight of silica in the composite particle was 22.1% by weight.

### (Example 15)

In the same manner as that of Example 14 except that, as the inorganic thickener, 4 g of KUNIBIS-110 (Kunimine Industries Co., Ltd. organic bentonite) was used, a silica-including microcapsule resin particle was obtained. The viscosity of a mixture was 1.01 mPa·s.

A cross section photograph of the resulting composite particle is shown in Fig. 17. It could be confirmed that an outer shell derived from a monofunctional monomer and a crosslinkable monomer is formed, and in the interior thereof, a porous structure in which silica particles are interconnected is formed. In addition, a volume average particle diameter was 13.5 µm, and the weight of silica in the composite particle was 21.9% by weight.

### (Comparative Example 3)

80 g of methyl methacrylate (MMA) as a monofunctional monomer, 80 g of ethylene glycol dimethacrylate (EGDMA) as a crosslinkable monomer, 128 g of tetraethoxysilane (TEOS) as a silica precursor, 32 g of toluene as an organic solvent, 1.6 g of 2,2'-azobis(2,4-dimethylvaleronitrile) (manufactured by Wako Pure Chemical Industries, Ltd.; product name V-65) as a polymerization initiator, and 0.8 g of (1R,2S,5R)-5-methyl-2-(propan-2-yl)cyclohexyl 4-methylbenzenesulfonate (manufactured by Wako Pure Chemical Industries, Ltd.; product name WPAG-699) as a thermal acid generator were mixed and dissolved to prepare a mixture.

Separately, in 1260 g of ion-exchanged water, 20 g of polyvinyl alcohol (PVA) (manufactured by The Nippon Synthetic Chemical Industry Co., Ltd.; product name GOHSENOL GL-05) as a suspension stabilizer was dissolved to prepare an aqueous phase. Into this aqueous phase, the above-mentioned mixture was placed, and emulsification/dispersion treatment for 3 minutes was performed at a rotation number of 5000 rpm, using a homogenizer (manufactured by CENTRAL SCIENTIFIC COMMERCE, INC.; product name Polytron Homogenizer PT10-35).

The resulting emulsified liquid was placed into a 2L pressure container with a stirring blade, heating for 4 hours was performed at 50°C while stirring the stirring blade at 350 rpm, to thereby form an outer shell composed of a monofunctional monomer and a crosslinkable monomer. While maintaining stirring, a reaction temperature was raised to 105°C, heating was performed for 2 hours, and thereby, a gelling reaction of TEOS was allowed to proceed. Thereafter, by lowering the reaction system to room temperature, an emulsified liquid containing a composite particle was obtained.

The resulting composite particle was taken out from the emulsified liquid by subjecting to centrifugation and separation of the supernatant, water washing was repeated, purification was performed, and thereafter, drying was performed in a vacuum oven at 60°C.

A surface photograph of the resulting composite particle is shown in Fig. 18 (A), and a cross section photograph is shown in Fig. 18 (B). It could be confirmed that an outer shell composed of a monofunctional monomer and a crosslinkable monomer is formed, and in the interior thereof, a porous structure in which silica particles are interconnected is formed. In addition, a volume average particle diameter was 10.4 µm, and the weight of a porous structure in the composite particle was 17.2% by weight.

Raw material species of the above-mentioned Examples 5 to 15 and Comparative Example 3, use amounts thereof (g), and the viscosity of the mixture are described together in Table 2.

**[Table 2]**

| | | | Example | | | | | | | | | | | Comparative Example |
|---|---|---|---|---|---|---|---|---|---|---|---|---|---|---|
| | | | 5 | 6 | 7 | 8 | 9 | 10 | 11 | 12 | 13 | 14 | 15 | 3 |
| Polymerizable composition | Monofunctional monomer | MMA | 80 | | | | | | | | | 100 | | 80 |
| | Crosslinkable monomer | EGDMA | 80 | | | | | | | | | 100 | | 80 |
| | Silica precursor | TEOS | 160 | | | | | | | | | 200 | | 128 |
| | Hydrophobic silica particle | R972 | 16 | 12 | 8 | 1.6 | 24 | 36 | | | | | | |
| | | R974 | | | | | | | 8 | | | | | |
| | | R976S | | | | | | | | 8 | | | | |
| | | R812 | | | | | | | | | 8 | | | |
| | Inorganic thickener | Sumecton-STN | | | | | | | | | | 4 | | |
| | | KUNIBIS-110 | | | | | | | | | | | 4 | |
| | Polymerization initiator | V-65 | 1.6 | | | | | | | | | 2 | | 1.6 |
| | Thermal acid generator | WPAG-699 | 0.8 | | | | | | | | | | | 0.8 |
| | Organic solvent | Toluene | | | | | | | | | | | | 32 |
| Viscosity of mixture (mPa·s) | | | 3.58 | 2.39 | 1.61 | 0.93 | 8.07 | 46.3 | 1.76 | 1.54 | 1.27 | 0.91 | 1.01 | |
| Aqueous phase | Ion-exchanged water | | 1280 | | | | | | | | | 1140 | | 1260 |
| | Magnesium pyrophosphate | | 26 | | | | | | | | | | | |
| | Lauryl phosphoric acid | | 0.128 | | | | | | | | | | | |
| | Polyvinyl alcohol | | | | | | | | | | | 60 | | 20 |

### (Assessment of Reflection Property of Ultraviolet, Visible, and Near Infrared Lights)

The reflectivities for ultraviolet light, visible light, and near infrared light of the composite particle were assessed by the following procedure.

To 10 g of a commercially available aqueous paint (ASAHIPEN CORPORATION, product name; Water-based multi use color, Clear), 2.5 g of the composite powder was added, and the mixture was stirred well to disperse the particle, to prepare paint for assessment.

The paint for assessment was coated on a black side of an opacifying ratio test paper with an applicator set at the wet thickness of 250 µm, thereafter, this was dried sufficiently under room temperature, and a sample plate for assessing the light reflectivity was obtained.

The reflectivities for ultraviolet light, visible light, and near infrared light of the sample plate were assessed by the following procedure.

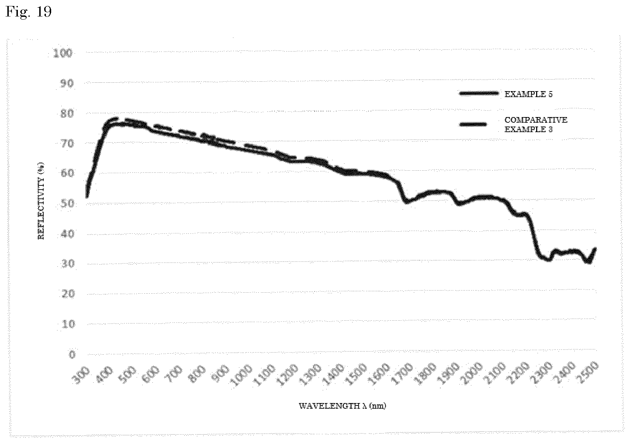

Using a ultraviolet, visible, and near infrared spectrophotometer (Solid Spec3700) manufactured by Shimadzu Corporation as an apparatus for measuring for reflectivity, the reflection property of from ultraviolet light to near infrared light (wavelength 300 to 2500 nm) of a coated surface in the sample plate was measured as the reflectivity (%). In addition, measurement was performed using a 60 mm φ integrating sphere, and employing Spectralon as a standard white plate. The obtained results are shown in Fig. 19.

From Fig. 19, it is seen that composite particles of Examples have high reflectivity in almost all wavelengths of from ultraviolet light to near infrared light, which is comparable to that of Comparative Example 3, without using an organic solvent like toluene.

### (Producing Example 1 of Paint Composition)

2 parts by weight of the composite particle obtained in Example 1, and 20 parts by weight of a commercially available acryl-based aqueous glossy paint (manufactured by Kanpe Hapio Co., Ltd., product name Super Hit) were mixed for 3 minutes, and defoamed for 1 minute using a stirring defoaming device, and thereby, a paint composition was obtained.

The resulting paint composition was coated on an ABS resin (acrylonitrile-butadiene-styrene resin) plate using a coating device set with a blade having the clearance of 75 µm, and thereafter, this was dried to thereby obtain a coated film.

### (Producing Example 2 of Paint Composition)

2 parts by weight of the composite particle obtained in Example 5, 20 parts by weight of a commercially available acryl-based aqueous glossy paint (manufactured by Kanpe Hapio Co., Ltd., product name Super Hit) were mixed for 3 minutes, and defoamed for 1 minute using a stirring defoaming device, and thereby, a paint composition was obtained.

The resulting paint composition was coated on an ABS resin (acrylonitrile-butadiene-styrene resin) plate using a coating device set with a blade having the clearance of 75 µm, and thereafter, this was dried to thereby obtain a coated film.

### (Producing Example 1 of Light Diffusing Resin Composition and Light Diffusion Film)

7.5 parts by weight of the composite particle obtained in Example 1, 30 parts by weight of an acrylic resin (manufactured by DIC Corporation, product name ACRYDIC A811), 10 parts by weight of a crosslinking agent (manufactured by DIC Corporation, product name VM-D), and 50 parts by weight of butyl acetate as a solvent were mixed for 3 minutes, and defoamed for 1 minute using a stirring defoaming device, and thereby, a light diffusing resin composition was obtained.

The resulting light diffusing resin composition was coated on a PET film having the thickness of 125 µm, using a coating device set with a blade having the clearance of 50 µm, thereafter, this was dried for 10 minutes at 70°C, and thereby, a light diffusion film was obtained.

### (Producing Example 2 of Light Diffusing Resin Composition and Light Diffusion Film)

7.5 parts by weight of the composite particle obtained in Example 5, 30 parts by weight of an acrylic resin (manufactured by DIC Corporation, product name ACRYDIC A811), 10 parts by weight of a crosslinking agent (manufactured by DIC Corporation, product name VM-D), and 50 parts by weight of butyl acetate as a solvent were mixed for 3 minutes, and defoamed for 1 minute using a stirring defoaming device, and thereby, a light diffusing resin composition was obtained.

The resulting light diffusing resin composition was coated on a PET film having the thickness of 125 µm using a coating device set with a blade having the clearance of 50 µm, thereafter, this was dried at 70°C for 10 minutes, and thereby, a light diffusion film was obtained.

### (Formulation Example of Cosmetic Material)

### (Blending Example 1)

### Producing of powder foundation

### ▪ Blending amount

| | |
|---|---|
| Composite particle obtained in Example 1 | 10.0 parts by weight |
| Red iron oxide | 3.0 parts by weight |
| Yellow iron oxide | 2.5 parts by weight |
| Black iron oxide | 0.5 part by weight |
| Titanium oxide | 10.0 parts by weight |
| Mica | 20.0 parts by weight |
| Talc | 44.0 parts by weight |
| Liquid paraffin | 5.0 parts by weight |
| Octyldodecyl myristate | 2.5 parts by weight |
| Vaseline | 2.5 parts by weight |
| Antiseptic | q. s. |
| Perfume | q. s. |

### ▪ Producing method

A composite particle, red iron oxide, yellow iron oxide, black iron oxide, titanium oxide, mica, and talc are mixed with a Henschel mixer, and to this is added one obtained by mixing and dissolving liquid paraffin, octyldodecyl myristate, vaseline, and an antiseptic, to uniformly mix them. To this is added perfume, and the materials are mixed, ground, and passed through a sieve. This is compression molded on a metal tray to obtain a powder foundation.

### (Blending Example 2)

### Producing of cosmetic milky lotion

### ▪ Blending amount

| | |
|---|---|
| Composite particle obtained in Example 1 | 10.0 parts by weight |
| Stearic acid | 2.5 parts by weight |
| Cetyl alcohol | 1.5 parts by weight |
| Vaseline | 5.0 parts by weight |
| Liquid paraffin | 10.0 parts by weight |
| Polyethylene (10 mole) monooleic acid ester | 2.0 parts by weight |
| Polyethylene glycol 1500 | 3.0 parts by weight |
| Triethanolamine | 1.0 part by weight |
| Purified water | 64.5 parts by weight |
| Perfume | 0.5 part by weight |
| Antiseptic | q. s. |

### ▪ Producing method

First, stearic acid, cetyl alcohol, vaseline, liquid paraffin, and polyethylene monooleic acid ester are heated and dissolved, a composite particle is added thereto, the materials are mixed, and a temperature is retained at 70°C (oily phase). Separately, polyethylene glycol and triethanolamine are added to purified water, the materials are heated and dissolved, and a temperature is retained at 70°C (aqueous phase). The oily phase is added to the aqueous phase, pre-emulsification is performed, thereafter, the pre-emulsion is uniformly emulsified with a homogenizer, and after emulsification, the emulsion is cooled to 30°C while mixing, to thereby obtain a cosmetic milky lotion.

### (Blending Example 3)

### Producing of powder foundation

### ▪ Blending amount

| | |
|---|---|
| Composite particle obtained in Example 5 | 10.0 parts by weight |
| Red iron oxide | 3.0 parts by weight |
| Yellow iron oxide | 2.5 parts by weight |
| Black iron oxide | 0.5 part by weight |
| Titanium oxide | 10.0 parts by weight |
| Mica | 20.0 parts by weight |
| Talc | 44.0 parts by weight |
| Liquid paraffin | 5.0 parts by weight |
| Octyldodecyl myristate | 2.5 parts by weight |
| Vaseline | 2.5 parts by weight |
| Antiseptic | q. s. |
| Perfume | q. s. |

### ▪ Producing method

A composite particle, red iron oxide, yellow iron oxide, black iron oxide, titanium oxide, mica, and talc are mixed with a Henschel mixer, and to this is added one obtained by mixing and dissolving liquid paraffin, octyldodecyl myristate, vaseline, and an antiseptic, to uniformly mix them. To this is added perfume, and the materials are mixed, ground, and passed through a sieve. This is compression molded on a metal tray to obtain a powder foundation.

### (Blending Example 4)

### Producing of cosmetic milky lotion

### ▪ Blending amount

| | |
|---|---|
| Composite particle obtained in Example 5 | 10.0 parts by weight |
| Stearic acid | 2.5 parts by weight |
| Cetyl alcohol | 1.5 parts by weight |
| Vaseline | 5.0 parts by weight |
| Liquid paraffin | 10.0 parts by weight |
| Polyethylene (10 mole) monooleic acid ester | 2.0 parts by weight |
| Polyethylene glycol 1500 | 3.0 parts by weight |
| Triethanolamine | 1.0 part by weight |
| Purified water | 64.5 parts by weight |
| Perfume | 0.5 part by weight |
| Antiseptic | q. s. |

### ▪ Producing method

First, stearic acid, cetyl alcohol, vaseline, liquid paraffin, and polyethylene monooleic acid ester are heated and dissolved, a composite particle is added thereto, the materials are mixed, and a temperature is retained at 70°C (oily phase). Separately, polyethylene glycol and triethanolamine are added to purified water, the materials are heated and dissolved, and a temperature is retained at 70°C (aqueous phase). The oily phase is added to the aqueous phase, pre-emulsification is performed, thereafter, the pre-emulsion is uniformly emulsified with a homomixer, and after emulsification, the emulsion is cooled to 30°C while mixing, to thereby obtain a cosmetic milky lotion.

## Claims

1. An organic-inorganic composite particle comprising:
an outer shell composed of a crosslinked polymer; and
a cavity that is partitioned with said outer shell,
wherein the composite particle contains, inside said cavity, a porous structure in which silica particles as a first inorganic particle are interconnected, and a second inorganic particle other than a silica particle, and has a volume average particle diameter of 0.5 to 100 µm.

2. The organic-inorganic composite particle according to claim 1, wherein said second inorganic particle has a refractive index of 1.8 or more.

3. The organic-inorganic composite particle according to claim 1 or 2, wherein said second inorganic particle has a particle diameter of 0.001 to 3 µm, which is measured by a dynamic light scattering method.

4. The organic-inorganic composite particle according to any one of claims 1 to 3, wherein said first inorganic particle and second inorganic particle have 5 to 50% by weight based on the total weight of said organic-inorganic composite particle, and imparts a hollow structure to said cavity.

5. The organic-inorganic composite particle according to any one of claims 1 to 4, wherein said second inorganic particle is a particle selected from titanium oxide, zirconium oxide, cerium oxide, zinc oxide, niobium oxide, and zirconium silicate.

6. A cosmetic material comprising the organic-inorganic composite particle according to any one of claims 1 to 5.

7. A paint composition comprising the organic-inorganic composite particle according to any one of claims 1 to 5.

8. A heat insulating resin composition comprising the organic-inorganic composite particle according to any one of claims 1 to 5.

9. A light diffusing resin composition comprising the organic-inorganic composite particle according to any one of claims 1 to 5.

10. A light diffusion film comprising the organic-inorganic composite particle according to any one of claims 1 to 5.

11. A method for producing an organic-inorganic composite particle, the method being a method for producing the organic-inorganic composite particle according to any one of claims 1 to 5, the method comprising steps of:
forming an outer shell composed of a crosslinked polymer, and a cavity that is partitioned with said outer shell, by suspension polymerizing a mixture comprising 100 parts by weight of a radical polymerizable monofunctional monomer, 20 to 150 parts by weight of a crosslinkable monomer, 60 to 400 parts by weight of silicon alkoxide as a silica precursor, and 0.1 to 10 parts by weight of a second inorganic particle, in presence of a radical polymerization initiator, in an aqueous medium; and
forming a porous structure in which silica particles are interconnected inside said cavity, by gelling silicon alkoxide after formation of said outer shell or simultaneously with formation of said outer shell.

12. The method for producing an organic-inorganic composite particle according to claim 11, wherein said gelling is performed using, as a catalyst, an acid or a base in a cavity that is partitioned with said outer shell, said acid or base is generated by external stimulation of a latent pH adjusting agent by energy radiation or heat, and said latent pH adjusting agent exists in said cavity, by dissolving said latent pH adjusting agent in a mixture at said suspension polymerization.

13. A method for producing an organic-inorganic composite particle, comprising steps of:
forming an outer shell composed of a crosslinked polymer, by suspension polymerizing a mixture comprising a radical polymerizable monofunctional monomer and crosslinkable monomer, silicon alkoxide as a silica precursor, and an inorganic thickener, in presence of a radical polymerization initiator and in absence of an organic solvent, in an aqueous medium; and
forming a porous structure in which silicon particles are interconnected inside said outer shell, from silicon alkoxide, after formation of said outer shell or simultaneously with formation of said outer shell.

14. The method for producing an organic-inorganic composite particle according to claim 13, wherein said mixture has a viscosity of 0.90 mPa·s or more at 25°C.

15. The method for producing an organic-inorganic composite particle according to claim 13 or 14, wherein said inorganic thickener is silicic anhydride or a clay mineral.

16. The method for producing an organic-inorganic composite particle according to any one of claims 13 to 15, wherein said porous structure in which silica particles are interconnected shows inclusion of a carbon component in EDX measurement.

17. The method for producing an organic-inorganic composite particle according to claim 15 or 16, wherein said inorganic thickener is a hydrophobic silica particle that is silicic anhydride, and said hydrophobic silica particle has a specific surface area by a BET method of 15 to 330 m²/g.

18. The method for producing an organic-inorganic composite particle according to any one of claims 13 to 17, wherein said organic-inorganic composite particle has a volume average particle diameter of 0.5 to 100 µm.

19. The method for producing an organic-inorganic composite particle according to any one of claims 13 to 18, wherein said porous structure has 5 to 50% by weight based on the total weight of said organic-inorganic composite particle.

20. The method for producing an organic-inorganic composite particle according to any one of claims 17 to 19, wherein said hydrophobic silica particle is contained in said mixture at 0.5 to 100 parts by weight, based on 100 parts by weight of said mixture.

21. The method for producing an organic-inorganic composite particle according to any one of claims 13 to 20, wherein said mixture comprises 100 parts by weight of said monofunctional monomer, 20 to 150 parts by weight of said crosslinkable monomer, and 60 to 400 parts by weight of said silica precursor.
